# EUROPEAN PATENT APPLICATION

(11) **EP 4 585 248 A1**
(43) Date of publication of application: **16.07.2025**
(21) Application number: 23872341.5
(22) Date of filing: 26.09.2023
(51) Int. Cl.: A61M 25/00, A61M 25/10

(54) **MEDICAL ELONGATED BODY AND BALLOON CATHETER**

(30) Priority: 29.09.2022 JP 2022156483
(71) Applicant: Terumo Kabushiki Kaisha, Tokyo 151-0072 (JP)
(72) Inventor: MOTOSE, Yuji, Fujinomiya-shi, Shizuoka 4180015 (JP); KOINUMA, Naoki, Fujinomiya-shi, Shizuoka 418-0015 (JP); SUGIKI, Tsutomu, Fujinomiya-shi, Shizuoka 4180015 (JP)
(74) Representative: Casalonga
(86) International application number: PCT/JP2023/034941
(87) International publication number: WO 2024/071118

(57) **Abstract**

Provided is a medical elongated body in which a bonding strength between a catheter shaft and a distal member is increased while the flexibility of a distal portion is maintained.

A medical elongated body 1 includes a catheter shaft 10 that extends in an axial direction and is provided with an outer layer 11 and an inner layer 12, a distal member 20 that is fused to a distal side of the catheter shaft and is more flexible than the catheter shaft, in which in a cross-sectional view in the axial direction, a distal portion 12A of the inner layer intrudes in an arc outward in the radial direction from an inner surface of the distal member, and
a distal portion 11A of the outer layer intrudes in an arc inward in the radial direction from an outer surface of the distal member.

## Description

### Technical Field

The present invention relates to a medical elongated body and a balloon catheter.

### Background Art

When carrying out various medical procedures inside living organs, a medical elongated body provided with a catheter shaft (shaft) that includes a hollow member with a lumen, a balloon catheter (for example, Patent Literature 1 below) including a shaft with a guide wire lumen, or the like may be used. In general, in the medical elongated body or the balloon catheter, an elongated shaft and a distal member disposed on the distal side of the shaft are provided.

As a method of bonding the shaft and the distal member, fusion is employed so that sufficient bonding strength is ensured between the shaft and the distal member. In this case, in order to obtain sufficient bonding strength, a long fusion length along an axial direction is required.

### Citation List

### Patent Literatures

Patent Literature 1: JP 2016-174827 A

### Summary of Invention

### Technical Problem

However, there is still room for various improvements other than increasing the fusion length along the axial direction.

An object of the present invention is to provide a medical elongated body and a balloon catheter, which have been made in consideration of the above problems.

### Solution to Problem

The above-described object of the present invention is achieved by the following means.

(1) A medical elongated body including:
   a catheter shaft that extends in an axial direction and is provided with an outer layer and an inner layer that is disposed inward in a radial direction of the outer layer;
   a distal member that is fused to a distal side of the catheter shaft and is more flexible than the catheter shaft, in which
   in a cross-sectional view in the axial direction,
   a distal portion of the inner layer intrudes in an arc outward in the radial direction from an inner surface of the distal member, and
   a distal portion of the outer layer intrudes in an arc inward in the radial direction from an outer surface of the distal member.
(2) A medical elongated body including:
   a catheter shaft that extends in an axial direction and is provided with an outer layer and an inner layer that is disposed inward in a radial direction of the outer layer; and
   a distal member that is fused to a distal side of the catheter shaft and is more flexible than the catheter shaft, in which
   in a cross-sectional view in the axial direction,
   a distal portion of the inner layer intrudes in an arc outward in the radial direction from an inner surface of the distal member, and
   the distal member includes an interposed portion that is interposed between a distal end of the inner layer and a distal end of the outer layer.
(3) A balloon catheter including:
   a shaft that extends in an axial direction;
   a distal member that is fused to a distal side of the shaft and is more flexible than the shaft; and
   a balloon that is disposed on an outer periphery of the shaft, in which
   the shaft includes
   an outer layer and
   an inner layer that is disposed inward in a radial direction of the outer layer,
   in a cross-sectional view in the axial direction,
   a distal portion of the shaft intrudes in an arc outward in the radial direction from an inner surface of the distal member, and
   a distal portion of the balloon intrudes in an arc inward in the radial direction from an outer surface of the distal member.
(4) The balloon catheter according to (3), in which
   the shaft includes
   an outer tube shaft that is provided with a lumen, and
   an inner tube shaft that is disposed in the lumen of the outer tube shaft.
(5) The balloon catheter according to (4), in which a thickness at a distal end of the balloon positioned at a portion fused to the inner tube shaft is equal to or smaller than a thickness of the inner tube shaft at a site closer to the proximal side than a portion where the balloon is bonded to the inner tube shaft, and is larger than a thickness of the inner tube shaft at a portion bonded to the balloon.
(6) The balloon catheter according to any one of (3) to (5), in which
   in the cross-sectional view in the axial direction,
   the distal portion of the balloon includes
   a wedge portion that is wedge-shaped, and
   the wedge portion includes a parallel portion parallel to the axial direction.
(7) The balloon catheter according to any one of (3) to (6), in which in the cross-sectional view in the axial direction, an inner surface-side proximal portion is provided in a section interposed between the inner surface of the distal member and an inner surface of the shaft in contact with the distal member, at least a part of a proximal portion of the distal member is positioned between the distal portion of the balloon and the distal portion of the shaft, and the distal portion of the shaft is positioned between the at least a part of the proximal portion of the distal member and the inner surface-side proximal portion.
(8) The balloon catheter according to any one of (3) to (7), in which in the cross-sectional view in the axial direction, the distal member is disposed to wrap around a distal end of the distal portion of the balloon.
(9) The balloon catheter according to any one of (3) to (8), in which the distal member and the balloon contain a polyamide elastomer.
(10) A medical elongated body comprising:
   a catheter shaft that extends in an axial direction and is provided with an outer layer and an inner layer that is disposed inward in a radial direction of the outer layer; and
   a distal member that is fused to a distal side of the catheter shaft, wherein
   in a cross-sectional view in the axial direction,
   a distal portion of the inner layer extends to be curved, along a distal portion of the outer layer, outward in a radial direction and toward a distal side from a site of a lumen of the catheter shaft as a starting point to a most distal end, and extends to be curved outward in the radial direction and toward a proximal side from the most distal end.
(11) A balloon catheter including:
   a shaft that extends in an axial direction;
   a distal member that is fused to a distal side of the shaft and is more flexible than the shaft; and
   a balloon that is disposed on an outer periphery of the shaft, in which
   the shaft includes
   an outer layer and
   an inner layer that is disposed inward in a radial direction of the outer layer,
   in a cross-sectional view in the axial direction,
   a distal portion of the shaft intrudes in an arc outward in the radial direction from an inner surface of the distal member,
   an outer peripheral surface of the distal portion of the shaft forms an outer periphery of the balloon catheter, and
   a most distal portion of the balloon is positioned inward in the radial direction with respect to an outer surface of the distal portion of the shaft.
(12) The balloon catheter according to (11), in which
   an inner surface-side proximal portion is provided in a section interposed between the inner surface of the distal member and an inner surface of the shaft in contact with the distal member, the inner surface-side proximal portion having a thickness in the section that increases in the radial direction along a curvature of the inner surface in a distal direction from a site of a lumen of the inner layer of the shaft, and
   an outer surface-side proximal portion is provided in a section interposed between an outer surface of the distal member and an outer surface of the shaft, the outer surface-side proximal portion having a thickness in the section that increases in the radial direction along a curvature of an outer surface of the outer layer in the distal direction from a site of the outer surface of the shaft.
(13) A medical elongated body including:
   a catheter shaft that extends in an axial direction and is provided with an outer layer and an inner layer that is disposed inward in a radial direction of the outer layer;
   a distal member that is fused to a distal side of the catheter shaft, in which
   in a cross-sectional view in the axial direction,
   a distal portion of the outer layer intrudes in an arc inward in the radial direction from an outer surface of the distal member, extends to be curved to a most distal end of the outer layer, is gradually tapered in a thickness, and terminates at an intermediate position in the radial direction of the catheter shaft.
(14) The medical elongated body according to (13), in which a distal portion of the inner layer intrudes in an arc outward in the radial direction from an inner surface of the distal member.

### Advantageous Effects of Invention

In the medical elongated body configured as described above, the distal portion of the inner layer intrudes in an arc outward in the radial direction from the inner surface of the distal member, and the distal portion of the outer layer intrudes in an arc inward in the radial direction from the outer surface of the distal member. Thus, the contact area between the inner layer and the distal member can be increased, and a sufficient bonding strength between the catheter shaft and the distal member can be obtained even though the fusion length is short. Therefore, it is possible to provide the medical elongated body in which the bonding strength between the catheter shaft and the distal member is increased while the flexibility of the distal portion is maintained.

Furthermore, in the medical elongated body configured as described above, the distal portion of the shaft intrudes in an arc outward in the radial direction from the inner surface of the distal member, and the distal portion of the balloon intrudes in an arc inward in the radial direction from the outer surface of the distal member. Thus, the contact area between the inner layer and the distal member can be increased, and a sufficient bonding strength between the shaft and the distal member can be obtained even though the fusion length is short. Therefore, it is possible to provide the balloon catheter in which the bonding strength between the shaft and the distal member is increased while the flexibility of the distal portion is maintained.

### Brief Description of Drawings

Fig. 1 is an overall configuration of a medical elongated body according to a first embodiment of the present invention.
Fig. 2 is a diagram illustrating a cross section along an axial direction of a distal portion of a medical elongated body according to a first embodiment.
Fig. 3 is a partially enlarged diagram of part A in Fig. 2.
Fig. 4 is a diagram for explaining a method of manufacturing a medical elongated body according to the first embodiment.
Fig. 5 is a diagram illustrating a cross section along an axial direction of a distal portion of a medical elongated body according to a second embodiment of the present invention.
Fig. 6 is a partially enlarged diagram of part A in Fig. 5.
Fig. 7 is a diagram illustrating an overall configuration of a balloon catheter according to a third embodiment of the present invention.
Fig. 8 is a diagram illustrating a cross section along an axial direction in the vicinity of a distal portion of a balloon catheter according to a third embodiment.
Fig. 9A is a partially enlarged diagram of the vicinity of the distal portion in Fig. 8.
Fig. 9B is a diagram of a balloon catheter according to a modification, which corresponds to Fig. 9A.
Fig. 10 is a diagram of a balloon catheter according to a modification of the third embodiment, which corresponds to Fig. 8.
Fig. 11 is a diagram of the balloon catheter according to the modification of the third embodiment, which corresponds to Fig. 9A.
Fig. 12 is a diagram of a balloon catheter according to a fourth embodiment, which corresponds to Fig. 9B.
Fig. 13 is a diagram of a balloon catheter according to a fifth embodiment, which corresponds to Fig. 9B.
Fig. 14 is an image diagram illustrating a state before molding of a balloon catheter according to a sixth embodiment.
Fig. 15 is a diagram of the balloon catheter according to the sixth embodiment, which corresponds to Fig. 9B.
Fig. 16 is an image diagram illustrating a state before molding of a balloon catheter according to a modification of the sixth embodiment.
Fig. 17 is a diagram of the balloon catheter according to the modification of the sixth embodiment, which corresponds to Fig. 9B.
Fig. 18 is a diagram of a balloon catheter according to a seventh embodiment, which corresponds to Fig. 9B.
Fig. 19 is a diagram of a balloon catheter according to Modification 1 of the seventh embodiment, which corresponds to Fig. 9B.
Fig. 20 is a diagram of a balloon catheter according to Modification 2 of the seventh embodiment, which corresponds to Fig. 9B.
Fig. 21 is a diagram of a balloon catheter according to an eighth embodiment, which corresponds to Fig. 9B.
Fig. 22 is a diagram of a medical elongated body according to a ninth embodiment, which corresponds to Fig. 3.

### Description of Embodiments

### <First Embodiment>

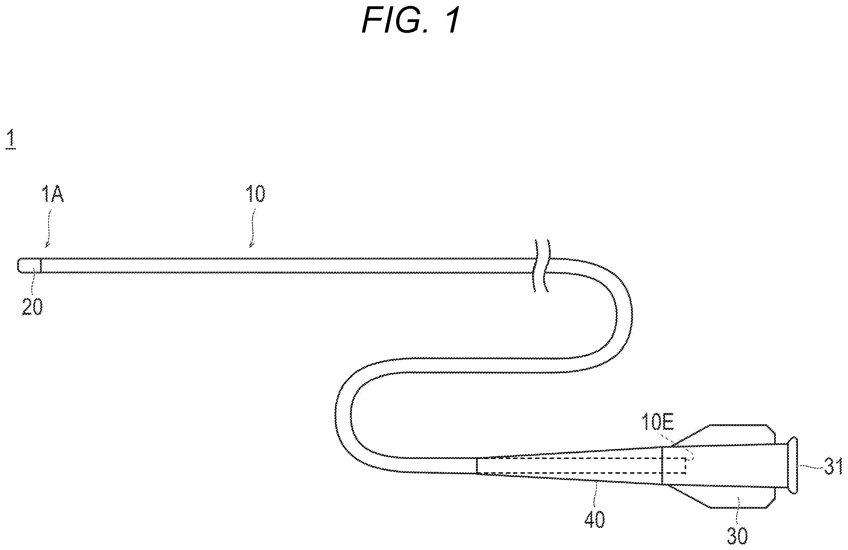

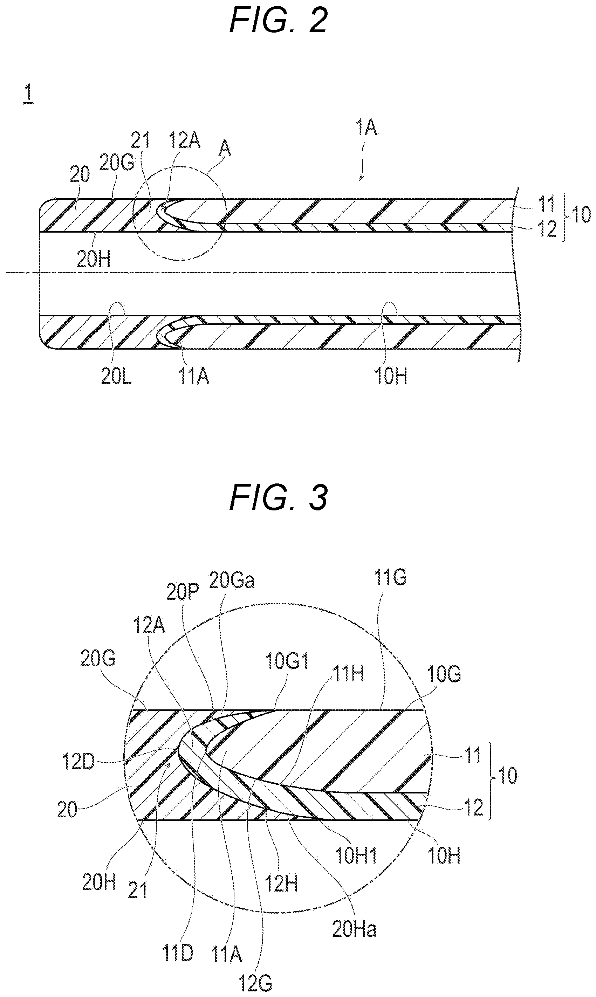

Hereinafter, a medical elongated body 1 according to a first embodiment will be described with reference to Figs. 1 to 3. Fig. 1 is an overall configuration of a medical elongated body 1 according to a first embodiment of the present invention. Fig. 2 is a diagram illustrating a cross section along an axial direction of a distal portion 1A of the medical elongated body 1 according to the first embodiment. Fig. 3 is a partially enlarged diagram of part A in Fig. 2. A dimensional ratio in each drawing is exaggerated for convenience of description, and may be different from an actual ratio.

In the present specification, a range from "X to Y" includes X and Y, and indicates "X or more and Y or less".

In a case where the configurations and the relationships between the configurations in the cross-sectional diagrams are described, the descriptions will be of the cross-sectional view in an axial direction unless otherwise specified.

The medical elongated body 1 is configured as a medical instrument such as a catheter or an endoscope inserted into a blood vessel, a bile duct, a trachea, an esophagus, a urethra, or other tubular cavity or lumen in a living body to perform treatment, diagnosis, or the like, or as a member of the medical instrument.

In the description of the first embodiment, the part (left side in Fig. 1) of the medical elongated body 1 that is inserted into a living body is referred to as a distal side, the part of the medical elongated body 1 on which a hub 30 is disposed is referred to as a proximal side, and the direction in which a catheter shaft 10 of the medical elongated body 1 extends is referred to as an axial direction. A distal portion represents a certain range including the distal end (the most distal end) and its periphery, while a proximal portion represents a certain range including the proximal end (the most proximal part) and its periphery.

As illustrated in Figs. 1 to 3, the medical elongated body 1 includes the catheter shaft 10 that extends in the axial direction, a distal member 20 that is disposed on the distal side of the catheter shaft 10, the hub 30 disposed on the proximal side of the catheter shaft 10, and a kink-resistant protector (strain relief) 40 disposed between the catheter shaft 10 and the hub 30.

As illustrated in Figs. 2 and 3, the catheter shaft 10 includes an outer layer 11 and an inner layer 12 disposed inward in the radial direction of the outer layer 11. The catheter shaft 10 and the distal member 20 are bonded to each other. Detailed configurations of the outer layer 11, the inner layer 12, and the distal member 20 will be described later.

As illustrated in Figs. 1 and 2, the hub 30 includes a port 31 that has a function as an insertion port through which a medical device such as a guide wire is inserted into the lumen 10H of the catheter shaft 10. The hub 30 can be attached to cover the outer periphery of a proximal portion 10E of the catheter shaft 10 using, for example, an adhesive, a fixture (not illustrated), or the like. Furthermore, examples of a constituent material for the hub 30 include a thermoplastic resin such as polycarbonate, polyamide, polysulfone, or polyarylate.

Hereinafter, the configurations of the outer layer 11, the inner layer 12, and the distal member 20 will be described in detail.

As illustrated in Fig. 2, the inner layer 12 is continuously formed along the axial direction of the catheter shaft 10. Specifically, the inner layer 12 extends along an inner peripheral surface of the outer layer 11 over substantially the entire length in the axial direction of the catheter shaft 10. For example, it is possible to use the catheter shaft 10 obtained by processing a material forming the inner layer 12 and a material forming the outer layer 11 into a hollow tubular shape having a layer structure by a known method such as co-extrusion molding.

As illustrated in Figs. 2 and 3, in the cross-sectional view in the axial direction, a distal portion 12A of the inner layer 12 is formed to intrude in an arc outward in the radial direction (upward in Fig. 3) from an inner surface 20H of the distal member 20. In other words, in the cross-sectional view in the axial direction as illustrated in Fig. 3, the distal portion 12A of the inner layer 12 extends to be curved outward in the radial direction and toward the distal side (left side in Fig. 2) of the medical elongated body 1 from a lumen 10H of the catheter shaft 10 as a starting point to a most distal end 12D, and extends to be curved outward in the radial direction and toward the proximal side (right side in Fig. 2) of the medical elongated body 1 from the most distal end 12D. More specifically, as illustrated in Fig. 3, the distal portion 12A of the inner layer 12 extends to be curved outward in the radial direction and toward the distal side (left side in Fig. 3) of the medical elongated body 1 from a site 10H1 of the lumen 10H of the catheter shaft 10 as the starting point to the most distal end 12D. An outer surface 12G of the inner layer 12 extends to be curved to the most distal end 11D along an edge of an inner surface 11H of the outer layer 11 while being in close contact with the inner surface 11H. The distal portion 12A of the inner layer 12 extends to be curved outward in the radial direction and toward the proximal side (right side in Fig. 3) of the medical elongated body 1 from the most distal end 12D. The outer surface 12G of the inner layer 12 extends along the edge of the inner surface 11H of the outer layer 11 while being in close contact with the inner surface 11H, and reaches an endpoint 10G1 of the outer periphery 10G. An inner surface 12H of the inner layer 12 extends along an edge of a proximal surface 20P of the distal member 20 while being in close contact with the proximal surface 20P in a melted state, and reaches the endpoint 10G1 of the outer periphery 10G. An inner surface-side proximal portion 20Ha of the distal member 20 is positioned in a section interposed between the inner surface 20H of the distal member 20 and the inner surface 12H of the inner layer 12 in contact with the proximal surface 20P of the distal member 20. The thickness of the inner surface-side proximal portion 20Ha in the section increases along a curvature of the inner surface 12H from the site 10H1 of the lumen 10H of the catheter shaft 10 toward a distal direction. An outer surface-side proximal portion 20Ga of the distal member 20 is positioned in a section interposed between an outer surface 20G of the distal member 20 and the inner surface 12H of the inner layer 12. The thickness of the outer surface-side proximal portion 20Ga in the section increases along a curvature of the inner surface 12H from a site 10G1 of the outer periphery 10G of the catheter shaft 10 toward the distal direction. The site 10G1 of the outer surface-side proximal portion 20Ga of the distal member 20 is positioned on the distal side in the axial direction with respect to the site 10H1 of the inner surface-side proximal portion 20Ha. The outer surface 20G of the distal member 20 and an outer surface 11G of the outer layer 11 form a straight portion without a level difference in the cross-sectional view in the axial direction. The inner surface 20H of the distal member 20 and the inner surface 12H of the inner layer 12 facing the lumen 10H form a straight portion without a level difference in the cross-sectional view in the axial direction. In the catheter shaft 10, the straight portion on the outer periphery and the straight portion on the inner periphery are parallel.

The inner surface-side proximal portion 20Ha and outer surface-side proximal portion 20Ga of the distal member 20 are positioned to sandwich the distal portion 12A of the inner layer 12 in the radial direction. The inner surface-side proximal portion 20Ha and outer surface-side proximal portion 20Ga of the distal member 20 are positioned to sandwich the most distal end 11D of the outer layer 11 in the radial direction. With the above-described configuration, even though the distal member 20 is excessively curved, the inner surface-side proximal portion 20Ha and the outer surface-side proximal portion 20Ga disperse stress to facilitate the release of detachment force, so that the detachment of the distal member 20 is minimized.

In the present embodiment, the most distal end of the medical elongated body 1 is the distal member 20. However, an embodiment in which another member is provided on the distal side of the distal member 20 is also adopted as a modification of the present embodiment.

The crystallinity of the inner layer 12 decreases from the proximal side toward the distal side. According to this configuration, the bending strength can be improved while the flexibility is maintained in the vicinity of the fusion portion on the distal member 20 side.

Crystallinity is an index indicating a degree of crystallinity of a resin, and is represented by (crystalline band intensity (absorbance)/amorphous band intensity (absorbance)) × 100 (%) in the present specification. The crystalline band refers to a band that disappears as confirmed by FT-IR measurement at a melting temperature, and the amorphous band refers to a band that does not disappear as confirmed by FT-IR measurement even at a melting temperature. Here, in a case where there are a plurality of crystalline bands and amorphous bands, one crystalline band and one amorphous band are selected as described below. Specifically, each peak with minimal overlap and baseline drift is selected. Furthermore, regarding the crystalline band intensity and the amorphous band intensity, for example, in a case where the resin is a polyamide-based resin (resin containing polyamide or polyamide elastomer), the crystalline band intensity is a crystalline band defined at an intensity of 1161 cm⁻¹. Moreover, for example, in a case where the resin is a polyamide-based resin (resin containing polyamide or polyamide elastomer), the amorphous band intensity is an amorphous band intensity defined at an intensity of 1369 cm⁻¹.

The crystallinity of the resin is measured as a crystallinity distribution confirmed by imaging IR. In the present specification, the measurement of the crystallinity distribution is carried out under the conditions as follows.
Measurement method: Microscopic-transmission method
Measurement range: 700 µm square (128 pixel square)
Element size: 5.5 µm square
Integration: 128 times
Spectral resolution: 4 cm⁻¹
Pretreatment: A sample is embedded in an epoxy resin and processed with a microtome into a thin section having a thickness of about 10 µm.

Furthermore, in the present specification, melting means that at least part of a resin is melted, preferably 80% by mass or more of the resin is melted, more preferably 95% by mass or more of the resin is melted, and still more preferably 99% by mass or more of the resin is melted. A melting temperature is not particularly limited as long as it is a temperature at which the resin melts, but in a case where the resin is a crystalline thermoplastic resin, the melting temperature is preferably a melting point +10°C or higher of the thermoplastic resin, and more preferably a melting point +20°C to 80°C of the thermoplastic resin. In a case where the resin is an amorphous thermoplastic resin, the melting temperature is preferably a glass transition temperature +10°C or higher of the thermoplastic resin, and more preferably a glass transition temperature +20°C to 80°C of the thermoplastic resin. Specifically, for example, the melting temperature is 150°C to 300°C, and 200°C to 250°C.

As illustrated in Figs. 2 and 3, in the cross-sectional view in the axial direction, a distal portion 11A of the outer layer 11 is formed to intrude in an arc inward in the radial direction (downward in Fig. 3) from the outer surface 20G of the distal member 20. In other words, the distal portion 11A of the outer layer 11 is formed in a convex shape so that the thickness is narrowed in the radial direction from the outer periphery 10G of the catheter shaft 10 toward the distal side. The most distal end 11D of the outer layer 11 is rounded. In a case where a perpendicular line is drawn along the axial direction so as to be brought into contact with the most distal end 11D of the outer layer 11, the distal member 20, the inner layer 12, the most distal end 11D of the outer layer 11, the inner layer 12, and the distal member 20 are positioned in this order in the radial direction from the center axis.

The distal member 20 is formed of a material that is more flexible than the catheter shaft 10. The distal member 20 is also referred to as a distal tip, and has a function of reducing damage to a lumen in a living body such as a blood vessel, a function of facilitating insertion into a branched blood vessel by following a guide wire that has already been inserted, and furthermore, a function of improving insertability into a stenosis occurring in the blood vessel.

As illustrated in Fig. 2, the distal member 20 has a tapered shape in which the outer diameter decreases toward the distal side. In the distal member 20, a through-hole 20L is formed to penetrate the distal member 20 in the axial direction. The through-hole 20L enables a medical device such as a guide wire inserted through the lumen 10H of the catheter shaft 10 to be guided out to the distal side of the medical elongated body 1.

The proximal portion 21 of the distal member 20 is formed in a concave shape to follow the shape of the distal portion 12A of the inner layer 12.

Examples of the resin forming the distal member 20 include polyolefins (for example, polyethylene, polypropylene, polybutene, ethylene-propylene copolymer, ethylene-vinyl acetate copolymer, ionomer, or a mixture of two or more types thereof), polyvinyl chloride, polyamide, polyamide elastomer, polyurethane, polyurethane elastomer, polyimide, and fluororesin. The above-described resin may be used singly or in combination of two or more types thereof. The resin forming the distal member is preferable to contain an elastomer having high flexibility and a high affinity and a small difference in hardness with an adjacent member, and more preferably a polyamide elastomer. The content of the polyamide elastomer in the resin contained in the distal member is preferably 50% by weight or more, more preferably 80% by weight or more, and still more preferably 100% by weight (consisting of a polyamide elastomer). The polyamide elastomer may be used singly or in combination of two or more types thereof.

The polyamide elastomer is a thermoplastic resin composed of a copolymer having a hard segment derived from a polymer that is crystalline and has a high melting point and a soft segment derived from a polymer that is amorphous and has a low glass transition temperature, the polyamide elastomer having amide bonds (-CONH-) in the polymer backbone forming the hard segment. Constituent units having amide bonds (-CONH-) in the polymer backbone forming the hard segment are also referred to as amide units of the polyamide elastomer.

The "amide units of the polyamide elastomer" in the present specification refers to repeating units derived from amide bonds in the polymer chain of the polyamide elastomer, and the amide units in the polyamide elastomer are preferably at least one of repeating units represented by Formula (1) or Formula (2) below.

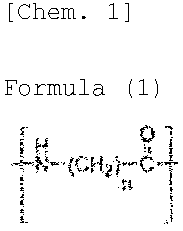

In Formula (1), n is preferably an integer of 2 to 20, and more preferably an integer of 5 to 11.

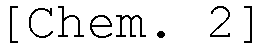

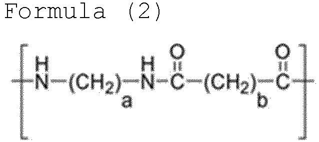

In Formula (2), a is preferably an integer of 4 to 12, b is preferably an integer of 4 to 10, a is more preferably an integer of 11 or 12, and b is more preferably an integer of 4 to 8.

Therefore, the polyamide elastomer preferably contains at least one of the repeating units represented by Formula (1) or Formula (2).

Examples of the polymer forming the soft segment include polyester and polyether. Moreover, examples thereof include polyethers such as polyethylene glycol, polypropylene glycol, polytetramethylene ether glycol (PTMG), and polyester polyol, and ABA-type triblock polyether diols. These can be used singly or in combination of two or more types thereof. Furthermore, a polyether diamine or the like obtained by reacting ammonia or the like with the terminal of polyether can be used, and for example, an ABA-type triblock polyether diamine can be used.

The content of the soft segment in the polyamide elastomer is preferably 1% to 50% by weight and more preferably 10% to 30% by weight.

Among these, the polyamide elastomer is preferably a polyether block amide copolymer in which a polyamide block as a hard segment and a polyether block as a soft segment are bonded via an ester bond.

The distal member can contain a pigment or dye that develops white, black, blue, red, or yellow, and a mixture thereof. Such a pigment or dye may be selected from materials that absorb a laser beam and generate heat. Examples of a material that absorbs a laser beam and generates heat (hereinbelow, also referred to as a laser beam-absorbing material) include carbon black, activated carbon, graphite, carbon nanotube, and fullerene; and condensed polycyclic organic pigments such as cyanine-based pigments, nickel dithiolene-based pigments, squarylium-based pigments, naphthoquinone-based pigments, diimmonium-based pigments, azo-based organic pigments, phthalocyanine-based pigments, naphthalocyanine-based pigments, and azulenocyanine-based pigments.

Furthermore, the distal member can be configured to include a powdered contrast medium. Specific examples of the material include compounds of gold, titanium, bismuth, and tungsten. Moreover, for the distal member, a reinforcing body made of tungsten, SUS, or the like may be disposed in the above-described material. Examples of a mode of the reinforcing body include a coil shape and a blade shape.

Examples of the resin constituting the catheter shaft 10 (outer layer 11 and inner layer 12) include polyolefins such as polypropylene, ethylene-propylene copolymer, and ethylene-vinyl acetate copolymer, thermoplastic resins such as modified polyolefin resin, soft polyvinyl chloride, various elastomers such as polyurethane elastomer, polyamide elastomer, and polyester elastomer, and crystalline plastics such as polyamide, crystalline polyethylene, and crystalline polypropylene.

Among these, the resin constituting the catheter shaft is preferably a polyamide and/or a polyamide elastomer. In a further suitable form, the resin constituting an outer layer 11 contains a polyamide elastomer, and the resin constituting an inner layer 12 contains a polyamide. In a further suitable form, the resin constituting the outer layer 11 contains a polyamide elastomer and a polyamide, and the resin constituting the inner layer 12 contains a polyamide. In a further suitable form, the resin constituting the outer layer 11 contains a polyamide elastomer and a polyamide, and the resin constituting the inner layer 12 is only composed of a polyamide. Since such a form is employed, the effects of the outer layer such as an improvement in abrasion resistance, a protection of the strength layer from external damage, and minimizing pinholes are easily exhibited, and the effects of the inner layer such as ensuring the required pressure resistance strength and compliance are also easily exhibited. Furthermore, since such a form is employed, the interlayer adhesion between the inner and outer layers of the shaft is improved.

Here, as the polyamide and/or the polyamide elastomer, a synthetic product or a commercially available product may be used.

For the inner layer of the catheter shaft, a modified polyolefin resin can also be used in addition to the polyamide-based resin described above. Examples of the modified polyolefin resin include polyethylene, polypropylene, an α-olefin (for example, 1-butene, 1-hexene, 4-methyl-1-pentene, 1-octene, 1-decene, and the like) polymer, an ethylene-propylene copolymer, a cycloolefin polymer (for example, a cyclic olefin polymer such as norbornene, cyclobutene, or cyclopentene), a cycloolefin copolymer (for example, a copolymer of a cyclic olefin and a chain olefin such as polyethylene, or a copolymer of a cyclic olefin and a diene such as 1,4-hexadiene), and mixtures thereof, which are materials with pendants containing polar and reactive groups; an ethylene-vinyl acetate copolymer. The copolymer has no particular limitation on the structure thereof and may be any one of a random copolymer, an alternating copolymer, a periodic copolymer, or a block copolymer. Among these, the modified polyolefin resin is preferably a modified polyethylene (such as high-density polyethylene, low-density polyethylene, or linear low-density polyethylene) or a modified polypropylene, and more preferably a modified polyethylene, from the viewpoint of the sliding properties of a modified polyolefin layer.

Examples of the polar group and reactivity group described above include a carboxyl group, a carboxylic anhydride group, a hydroxyl group, an alkoxy group, an imide group, an acryloyl group, a methacryloyl group, a silanyl group, and/or a silanol group.

As the modified polyolefin resin, a commercially available product may be used, and examples thereof include MODIC (registered trademark) series (H511, H503, L502, L533, L504, M142, M502, M512, M545, A543, A515), LINKLON (registered trademark) series (all of which are manufactured by Mitsubishi Chemical Corporation), Admer (registered trademark) series (manufactured by Mitsui Chemicals, Inc.), and HIMILAN (registered trademark) series (Du Pont-Mitsui Polychemicals Co., Ltd.).

The weight-average molecular weight of the modified polyolefin resin is preferably 1,000 to 10,000,000.

In a case where a modified polyolefin resin is used for the inner layer of the catheter shaft, the resin constituting the outer layer 11 preferably contains a polyamide and/or a polyamide elastomer. In a further suitable form, the resin constituting the outer layer 11 is preferably a polyamide and/or a polyamide elastomer. As another suitable form, the resin constituting the outer layer 11 preferably contains a polyamide and a polyamide elastomer. As another suitable form, it is preferable that a middle layer is provided between the inner layer containing the modified polyolefin resin and the outer layer, the middle layer and the outer layer contain a polyamide, and the outer layer contains a laser beam-absorbing material described later. Since such a form is employed, the effects of the outer layer such as an improvement in abrasion resistance, a protection of the strength layer from external damage, and minimizing pinholes are easily exhibited, and the effects of the inner layer such as ensuring the required pressure resistance strength and compliance are also easily exhibited. Furthermore, since such a form is employed, the interlayer adhesion between the inner and outer layers of the shaft is improved.

Furthermore, the catheter shaft (specifically, shaft inner layer) can contain a pigment or dye that develops white, black, blue, red, or yellow, and a mixture thereof. Such a pigment or dye may be selected from materials that absorb a laser beam and generate heat. Examples of a material that absorbs a laser beam and generates heat (laser beam-absorbing material) include carbon black, activated carbon, graphite, carbon nanotube, and fullerene; and condensed polycyclic organic pigments such as cyanine-based pigments, nickel dithiolene-based pigments, squarylium-based pigments, naphthoquinone-based pigments, diimmonium-based pigments, azo-based organic pigments, phthalocyanine-based pigments, naphthalocyanine-based pigments, and azulenocyanine-based pigments. In a suitable aspect, the catheter shaft inner layer contains a laser beam-absorbing material and the catheter shaft outer layer is substantially free of the laser beam-absorbing material. According to such an aspect, when the inner layer absorbs a laser beam and generates heat, the temperature of an inner layer resin increases to be higher than the glass transition point thereof, deformation associated with pressurization is accelerated, and furthermore, the temperature reaches the melting point to increase fluidity, and the form of "the distal portion of the inner layer intrudes in an arc outward in the radial direction from the inner surface of the distal member, and the distal portion of the outer layer intrudes in an arc inward in the radial direction from the outer surface of the distal member" is thus easily achieved. The blending amount of the laser beam-absorbing material is not particularly limited, but for example, 1% to 10% by weight, and may be 2% to 8% by weight in the inner layer. In a case where the laser beam-absorbing material is blended in the shaft inner layer, it is preferable to select a transparent shaft outer layer containing the resin described above. Accordingly, the inner layer easily achieves the above-described form.

As necessary, the surface of the outermost layer capable of coming into contact with a living body may be coated with a hydrophilic lubricating material. The hydrophilic lubricating material is not particularly limited as long as it is hydrophilic and has a lubricating property, and a known material can be used. Specific examples thereof include copolymers of epoxy group-containing monomers such as glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether with hydrophilic monomers such as N-methylacrylamide, N,N-dimethylacrylamide, and acrylamide; (co)polymers containing the above-described hydrophilic monomer; cellulose-based polymer substances such as hydroxypropyl cellulose and carboxymethyl cellulose; polysaccharides, polyvinyl alcohol, methyl vinyl ether-maleic anhydride copolymers, water-soluble polyamides, poly(2-hydroxyethyl (meth)acrylate), polyethylene glycol, polyacrylamide, polyvinylpyrrolidone, and copolymers of polyvinylpyrrolidone and polyurethane described in US 4,100,309 and JP S59-19582 A. These hydrophilic lubricating materials may be used singly or in the form of a mixture of two or more types thereof.

Furthermore, as necessary, the surface of the outer layer containing the polyamide elastomer formed on the outermost layer capable of coming into contact with a living body may be further coated with a biocompatible material or an antithrombotic material. As the biocompatible material and the antithrombotic material, various known polymers can be used singly or in combination, and for example, natural polymers (such as collagen, gelatin, chitin, chitosan, cellulose, polyaspartic acid, polyglutamic acid, and polylysine, casein) and synthetic polymers (such as phospholipid polymers, methacryloyloxyethyl phosphorylcholine (MPC) block polymers with a phosphate group in the side chain, polyhydroxyethyl methacrylate, copolymer of hydroxyethyl methacrylate and styrene (for example, HEMA-St-HEMA block copolymer), polymethyl methacrylate, polylactic acid, polyglycolic acid, lactic acid-glycolic acid copolymer, polyethylene, and polypropylene) can be suitably used.

Next, a method of manufacturing the medical elongated body 1 according to the present embodiment will be described.

The medical elongated body 1 according to the present invention is manufactured by bonding the proximal portion part of the distal member 20 and the distal portion part of the catheter shaft 10. Specifically, it is preferable to include the following steps of: (1) inserting a contact portion between a distal member 20 and a catheter shaft 10 into a hollow portion of an elastic body (step (A)), (2) performing pressurization inward in a radial direction and heating (step (B)), and (3) releasing the pressurization and taking out a fused (bonded) elongated body (step (C)).

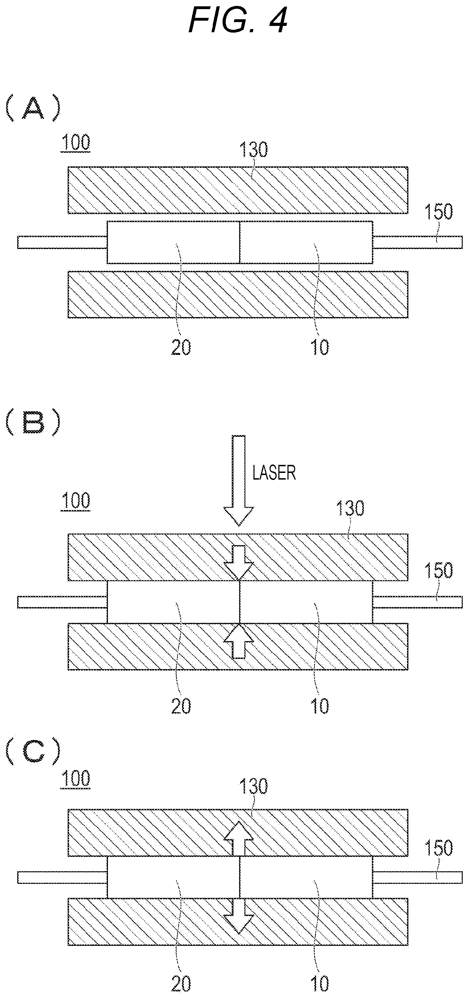

Fig. 4 is a diagram for explaining a method of manufacturing the medical elongated body 1 according to the first embodiment.

### (Step (A))

The catheter shaft 10 and the distal member 20 are inserted through a core material 150. As a result, the catheter shaft 10 and the distal member 20 can move and rotate integrally with the core material 150. The core material is made of, for example, metal.

Next, in a state where the rotation axes of the core material 150 and an elastic body 130 are aligned with each other, any one of the core material 150 or the elastic body 130 is moved in the axial direction to dispose the contact portion between the catheter shaft 10 and the distal member 20 in the hollow portion of the elastic body 130. In this state, the catheter shaft 10 and the distal member 20 are not in contact with the hollow portion of the elastic body 130 in the radial direction, and there is a gap. The elastic body has laser transmission properties, and specific examples of a material of the elastic body include silicone rubber and fluororubber. Since the elastic body is used, the elastic body can be elastically deformed in the radial direction by pressurization with a pressurizing member described later to maintain a contact state between the shaft and the distal member, thereby efficiently performing the bonding. Furthermore, since the elastic body has laser transmission properties, the elastic body is not thermally shrunk by laser beam, and thus can be reused.

### (Step (B))

Next, the contact portion between the catheter shaft and the distal member is pressurized in the radial direction. The pressurization method is not particularly limited, but for example, a hollow cylindrical pressurizing member can be used. Similar to the core material and the elastic body, the hollow cylindrical pressurizing member is configured to be rotatably supported with the axial direction as a rotation axis. Then, the elastic body can be inserted into the hollow portion of the pressurizing member having such a shape. In this case, the elastic body can be inserted into the hollow portion of the pressurizing member by extending the elastic body in the axial direction so that the diameter of the elastic body is smaller than the inner diameter of the pressurizing member. Moreover, after the elastic body is disposed in the hollow portion of the pressurizing member, the extension in the axial direction is released. The inner diameter of the pressurizing member is preferably smaller than the diameter (outer diameter) of the elastic body. Since the outer diameter of the elastic body and the inner diameter of the pressurizing member are designed in this manner, the elastic body comes into contact with the pressurizing member when the extension is released, and the external pressure is applied inward in the radial direction. The form of applying the external force is not limited to the above-described form, and for example, the elastic body may be pressurized in the radial direction by bringing a rectangular parallelepiped pressurizing member into contact with the elastic body in the radial direction.

As described above, since the pressure is applied using the elastic body and the pressurizing member during the heating without applying the pressure during the setup, it is possible to perform highly accurate alignment, and effects such as improvement in quality of fusion and improvement in reproducibility are exerted. Furthermore, since there is a clearance between a workpiece and the elastic body, fusion processing can be performed without affecting the workpiece when the workpiece is inserted into the elastic body. Moreover, imparting a desired shape to the elastic body or the pressurizing member in advance enables the fusion portion of the workpiece to be formed into a tapered shape, a two-stage tapered shape, a gentle round shape, a local constricted portion, or the like.

The shape of the pressurizing member is not limited to the hollow cylindrical shape, and is not particularly limited as long as it can pressurize the elastic body in the radial direction. The pressurizing member has laser transmission properties, similar to the elastic body. The term "laser transmission properties" means that the transmittance of the laser beam is 80% or more with respect to a thickness of 1 mm in the radial direction. A material used for the pressurizing member may be any material that can apply an external force to the elastic body, and examples thereof include glass, quartz, and sapphire.

Next, the contact portion is irradiated with the laser beam while the external force is applied. Since the pressurizing member and the elastic body have laser transmission properties, the contact portion is directly heated. Furthermore, the contact portion can be locally heated by locally irradiating the contact portion with the laser beam emission. The core material 150, the elastic body 130, and the pressurizing member (not illustrated) may rotate about the axial direction as a rotation axis during the heating to cause the distal member 20 and the catheter shaft 10 to be rotated about the axial direction as a rotation axis, so that the heating is uniformly performed.

The laser beam is locally emitted to end face portions and peripheral portions of the two members in a state where both members are arranged in the axial direction. Such laser beam emission causes heat generation of the catheter shaft 10 and/or the distal member 20 themselves and further causes heat transfer from the core material 150 to the catheter shaft 10 and the distal member 20, and the contact portion between the proximal side of the distal member 20 and the distal side of the catheter shaft 10 is thus fused to form a fusion portion. A laser beam-absorbing material is preferably contained in at least one of the catheter shaft 10 and/or the distal member 20, and preferably at least the catheter shaft 10 to be locally heated. Furthermore, since the peripheral elastic body has laser transmission properties, the elastic body is not heated, and heat is dissipated to the elastic body and the periphery (for example, core material) from the moment when the fusion portion is heated. It is considered that such localized rapid heating followed by rapid cooling makes it difficult for crystals to grow, and the crystallinity of the fusion portion thus decreases.

Regarding the laser beam emission, a laser beam having a wavelength that causes the fusion portion to generate heat by radiation heating is emitted. The spot diameter of the laser beam can be set to φ0.1 to φ10 mm, and the wavelength of the laser beam can be set to 800 to 10,000 nm. As the laser beam, a fiber laser (a wavelength of 1070 nm), a YAG laser (a wavelength of 1064 nm), a laser diode (808 nm, 840 nm, 940 nm), or the like can be used. In a case of fusion between the members in which the laser beam-absorbing material is blended, the wavelength of the laser beam can be selected from 800 to 5,000 nm, preferably 900 to 2,300 nm. In a case of fusion between the transparent members or between the member in which the laser beam-absorbing material is blended and the transparent member, the wavelength of the laser beam can be selected from 1,300 to 2,500 nm, preferably 1,500 to 2,300 nm. The laser beam is substantially emitted in a direction orthogonal to the axial direction of the workpiece. The laser beam may be emitted at an angle appropriately changed according to the workpiece.

Furthermore, in a case where heating is performed by laser emission while the pressurization is applied, and particularly a case where the shaft inner layer contains the laser beam-absorbing material, the shaft inner layer generates heat by a laser beam, and then reaches the melting point, resulting in an increase in fluidity. As a result, the shaft inner layer is easily pushed out toward the distal member side. It is considered that this series of configurations causes a shape in which the distal portion of the shaft inner layer intrudes in an arc outward in the radial direction from the inner surface of the distal member. Moreover, in the case where heating is performed by laser emission while the pressurization is applied, and particularly the case where the shaft inner layer contains the laser beam-absorbing material, the shaft outer layer is under the most pressure, and the distal end of the shaft is further tapered by heating, so that the inner layer and the outer layer are likely to be integrated.

The pressurization on the elastic body is finally released, and the medical elongated body formed by fusion can be taken out.

As described above, the medical elongated body 1 according to the first embodiment includes the catheter shaft 10 that extends in the axial direction and includes the outer layer 11 and the inner layer 12 disposed inward in the radial direction of the outer layer 11, and the distal member 20 that is fused to the distal side of the catheter shaft 10 and is more flexible than the catheter shaft 10. In the cross-sectional view in the axial direction, the distal portion 12A of the inner layer 12 intrudes in an arc outward in the radial direction from the inner surface of the distal member 20, and the distal portion 11A of the outer layer 11 intrudes in an arc inward in the radial direction from the outer surface of the distal member 20. According to the medical elongated body 1 configured in such a manner, the contact area between the inner layer 12 and the distal member 20 can be increased, and a sufficient bonding strength between the catheter shaft 10 and the distal member 20 can be obtained even though the fusion length is short. Therefore, it is possible to provide the medical elongated body 1 in which the bonding strength between the catheter shaft 10 and the distal member 20 is increased while the flexibility of the distal portion 1A of the medical elongated body 1 is maintained.

Furthermore, the crystallinity of the inner layer 12 decreases from the proximal side toward the distal side. According to the medical elongated body 1 configured in such a manner, the bending strength can be improved while the flexibility is maintained in the vicinity of the fusion portion on the distal member 20 side.

### <Second Embodiment>

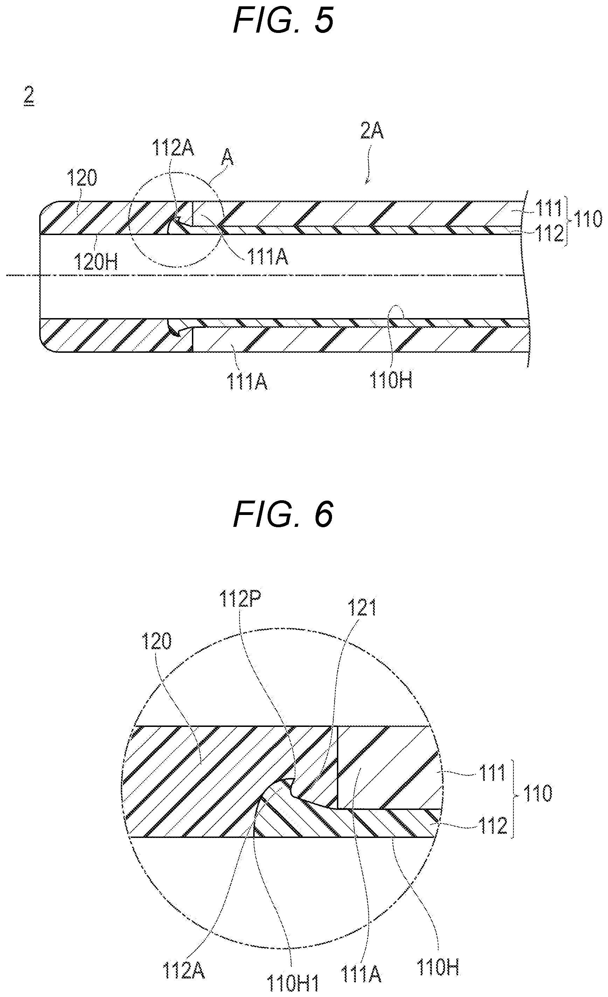

Next, a configuration of a medical elongated body 2 according to a second embodiment of the present invention will be described with reference to Figs. 5 and 6. Fig. 5 is a diagram illustrating a cross section along an axial direction of a distal portion 2A of the medical elongated body 2 according to the second embodiment. Fig. 6 is a partially enlarged diagram of part A in Fig. 5.

Configurations common to the first embodiment will not be described, and configurations characteristic of the second embodiment will be described. The same members as those of the first embodiment described above will be denoted by the same reference numerals, and the descriptions thereof will not be repeated. The second embodiment is different from the first embodiment in configurations and the like of a catheter shaft 110 and a distal member 120.

The medical elongated body 2 according to the second embodiment includes a catheter shaft 110 that extends in an axial direction, a distal member 120 that is disposed on the distal side of the catheter shaft 110, a hub 30 disposed on the proximal side of the catheter shaft 110, and a kink-resistant protector 40 disposed between the catheter shaft 110 and the hub 30.

Since the hub 30 and the kink-resistant protector 40 have the same configurations as those of the medical elongated body 1 according to the first embodiment described above, the descriptions thereof will not be repeated.

As illustrated in Figs. 5 and 6, the catheter shaft 110 includes an outer layer 111 and an inner layer 112 disposed inward in the radial direction of the outer layer 111. The catheter shaft 110 and the distal member 120 are bonded to each other.

As illustrated in Figs. 5 and 6, in the cross-sectional view in the axial direction, a distal portion 112A of the inner layer 112 is formed to intrude in an arc outward in the radial direction (upward in Fig. 6) from an inner surface 120H of the distal member 120. In other words, in the cross-sectional view in the axial direction, as illustrated in Figs. 5 and 6, the distal portion 112A of the inner layer 112 is configured to be curved outward in the radial direction from a starting point 110H1 of a lumen 110H of the catheter shaft 110 and toward the proximal side of the medical elongated body 2, and is configured as an engagement portion to be engaged with an interposed portion 121 of the distal member 120 described later.

In the cross-sectional view in the axial direction as illustrated in Figs. 5 and 6, the outer layer 111 is formed in a cylindrical shape such that the outer diameter and the inner diameter are substantially constant from the proximal end toward the distal end.

As illustrated in Figs. 5 and 6, the distal member 120 has the interposed portion 121 that is interposed between the distal portion 112A of the inner layer 112 and a distal portion 111A of the outer layer 111. The interposed portion 121 is engaged with the distal portion 112A of the inner layer 112. An apex portion 112P of the distal portion 112A further extends toward the proximal direction in the axial direction. As a result, the interposed portion 121 of the distal member 120 is interposed between the apex portion 112P of the distal portion 112A extending like a claw and the distal portion 111A of the outer layer 111, and the engagement between the distal member 120 and the catheter shaft 110 is strengthened. As a result, the distal member 120 is not easily detached from the catheter shaft 110 even when bent or stretched.

As described above, the medical elongated body 2 according to the second embodiment includes the catheter shaft 110 that extends in the axial direction and includes the outer layer 111 and the inner layer 112 disposed inward in the radial direction of the outer layer 111, and the distal member 120 that is fused to the distal side of the catheter shaft 110 and is more flexible than the catheter shaft 110. In the cross-sectional view in the axial direction, the distal portion 112A of the inner layer 112 intrudes in an arc outward in the radial direction from the inner surface of the distal member 120, and the distal member 120 has the interposed portion 121 that is interposed between the distal portion 112A of the inner layer 112 and the distal portion 111A of the outer layer 111. According to the medical elongated body 2 configured as described above, the distal portion 112A of the inner layer 112 and the interposed portion 121 are engaged with each other to improve the bonding strength between the inner layer 112 and the distal member 120, and a sufficient bonding strength between the catheter shaft 110 and the distal member 120 can thus be obtained even though the fusion length is short. Therefore, it is possible to provide the medical elongated body 2 in which the bonding strength between the catheter shaft 110 and the distal member 120 is increased while the flexibility of the distal portion 2A of the medical elongated body 2 is maintained.

### <Third Embodiment>

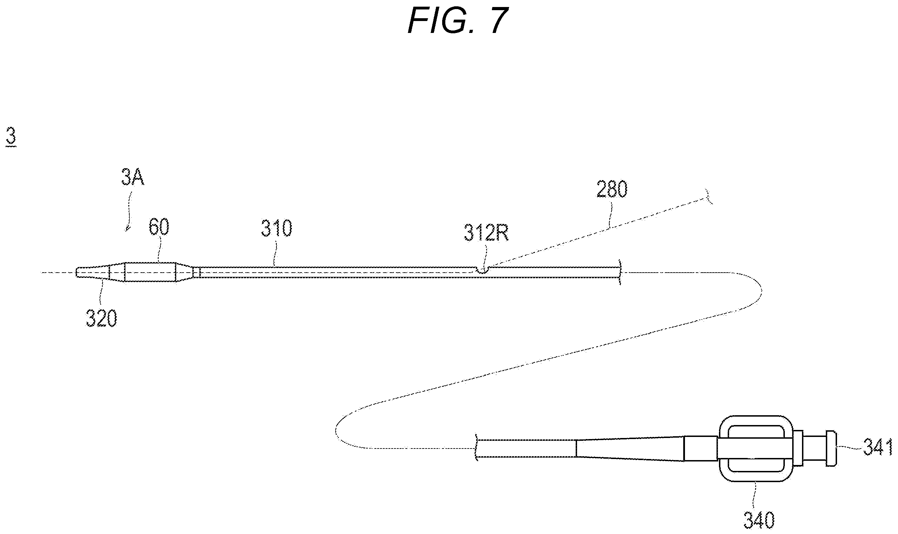

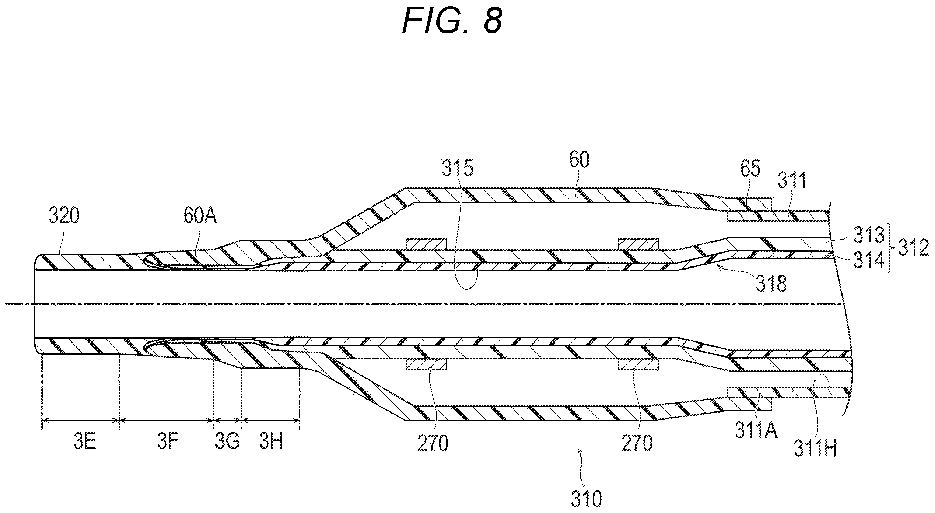

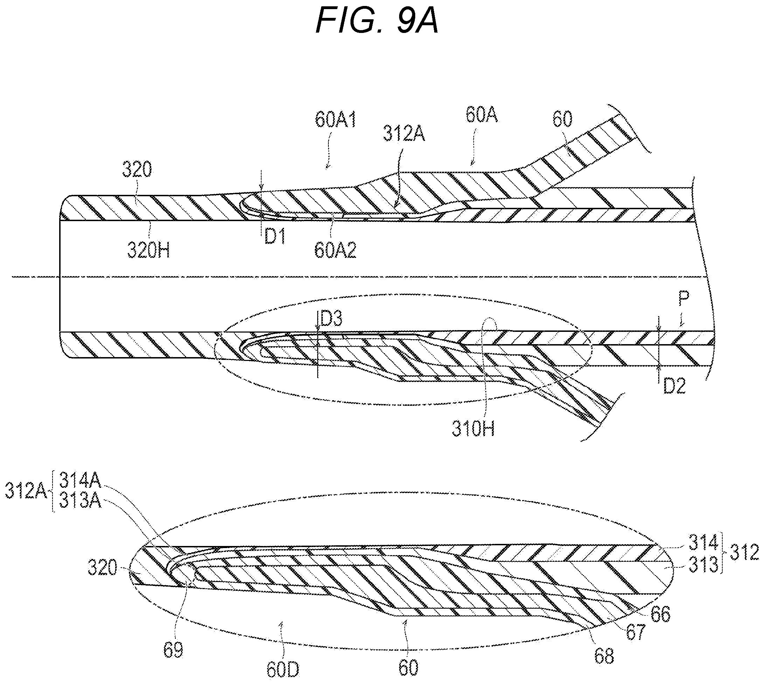

Next, a balloon catheter 3 according to a third embodiment of the present invention will be described with reference to Figs. 7 to 9A. Fig. 7 is a diagram illustrating an overall configuration of the balloon catheter 3 according to the third embodiment of the present invention. Fig. 8 is a diagram illustrating a cross section along the axial direction in the vicinity of a distal portion 3A of the balloon catheter 3 according to the third embodiment. Fig. 9A is a partially enlarged view at the distal portion 3A in Fig. 8.

The balloon catheter 3 according to the third embodiment is a medical device used to perform a treatment for a stenosis (lesion). In the treatment, an elongated shaft 310 is inserted into a biological organ, and a balloon 60 disposed on the distal side of the shaft 310 is expanded at the stenosis to push and widen the stenosis.

In the third embodiment, the balloon catheter 3 is configured as a balloon catheter for PTCA used to expand a stenosis of a coronary artery. However, a configuration for the purpose of treating and alleviating a stenosis developed in a biological organ such as another blood vessel, a bile duct, a trachea, an esophagus, another digestive tract, a urethra, an ear and nose lumen, or another organ can be adopted. Furthermore, a configuration as a delivery balloon catheter used for the purpose of transporting a medical instrument such as a stent in a living body can be adopted.

In the description of the third embodiment, the part (left side in Fig. 7) of the balloon catheter 3 that is inserted into a living body is referred to as a distal side, the part of the balloon catheter 3 on which a hub 340 is disposed is referred to as a proximal side, and the direction in which a shaft 310 of the balloon catheter 3 extends is referred to as an axial direction. A distal portion represents a certain range including the distal end (the most distal end) and its periphery, while a proximal portion represents a certain range including the proximal end (the most proximal part) and its periphery.

As illustrated in Fig. 7, the balloon catheter 3 includes the shaft 310 extending in the axial direction, a distal member 320 disposed on the distal side of the shaft 310, the hub 340 disposed on the proximal side of the shaft 310, and the balloon 60 disposed on the outer periphery of the shaft 310.

As illustrated in Fig. 8, the shaft 310 includes the outer tube shaft 311 provided with the lumen 311H, and the inner tube shaft 312 disposed in the lumen 311H of the outer tube shaft 311.

The shaft 310 has a double tube structure in which an inner tube shaft 312 and an outer tube shaft 311 are arranged with a concentric alignment. In the present embodiment, the balloon catheter 3 is of what is called a rapid exchange type provided with a proximal opening (proximal opening of inner tube shaft 312) 312R through which a guide wire 280 is guided out toward the distal portion part of the shaft 310. The inner tube shaft 312 and the outer tube shaft 311 may not be arranged with a concentric alignment. That is, the center axis of the inner tube shaft 312 and the center axis of the outer tube shaft 311 may be arranged to be shifted from each other.

As illustrated in Figs. 8 and 9A, the inner tube shaft 312 has a tubular shape in which a guide wire lumen 315 through which the guide wire 280 is inserted is formed.

A lumen 311H included in the outer tube shaft 311 has a function as a lumen for a pressurizing medium through which a pressurizing medium flows between the outer tube shaft 311 and the inner tube shaft 312.

The hub 340 includes a port 341 connectable in a liquid-tight and air-tight manner to a supplying device such as an indeflator (not illustrated) for supplying a pressurizing medium to the balloon 60. The port 341 of the hub 340 may be, for example, a known Luer taper which a fluid tube or the like can be connected to or separated from. The pressurizing medium (for example, saline, contrast medium, and the like) can flow into the lumen 311H of the outer tube shaft 311 via the port 341 of the hub 340, and is supplied to the balloon 60 through the lumen 311H.

As illustrated in Figs. 8 and 9A, the inner tube shaft 312 includes an outer layer 313 and an inner layer 314 disposed inward in the radial direction of the outer layer 313. As illustrated in Fig. 8, the inner tube shaft 312 has a tapered portion 318 whose diameter decreases from the proximal end toward the distal end in the vicinity of a proximal portion 65 of the balloon 60 in the axial direction. The outer diameter of the inner tube shaft 312 closer to the proximal side than the tapered portion 318 is larger than the outer diameter of the inner tube shaft 312 where X-ray radiopaque markers 270 described later are positioned, and the outer diameter of the inner tube shaft 312 where the X-ray radiopaque markers 270 are positioned is larger than the outer diameter of the inner tube shaft 312 at the portion where the balloon 60 is bonded to the inner tube shaft 312. The inner diameter of the inner tube shaft 312 closer to the proximal side than the tapered portion 318 is larger than the inner diameter of the inner tube shaft 312 where X-ray radiopaque markers 270 described later are positioned, and larger than the outer diameter of the inner tube shaft 312 at the portion where the balloon 60 is bonded to the inner tube shaft 312.

In the cross-sectional view in the axial direction as illustrated in Fig. 9A, a distal portion 312A of the inner tube shaft 312 is formed to intrude in an arc outward in the radial direction from an inner surface 320H of the distal member 320. In other words, as illustrated in Fig. 9A, the distal portion 312A of the inner tube shaft 312 extends to be curved outward in the radial direction and toward the distal end of the balloon catheter 3 from the lumen 310H of the shaft 310 to the distal end.

In the cross-sectional view in the axial direction, the distal portions 313A and 314A of the outer layer 313 and the inner layer 314 of the inner tube shaft 312 are formed to intrude in an arc outward in the radial direction from the inner surface 320H of the distal member 320. In other words, as illustrated in Fig. 9A, a distal portion 313A of the outer layer 313 and a distal portion 314A of the inner layer 314 extends to be curved outward in the radial direction and toward the distal end of the balloon catheter 3 from the lumen 310H of the shaft 310 to the distal end.

The crystallinity of the inner layer 314 of the inner tube shaft 312 decreases from the proximal side toward the distal side. According to this configuration, the bending strength can be improved while the flexibility is maintained in the vicinity of the fusion portion on the distal member 320 side.

As illustrated in Fig. 9A, the distal portion 312A of the inner tube shaft 312 has a wall thickness smaller than that of a portion other than the distal portion 312A because the balloon 60 intrudes therein.

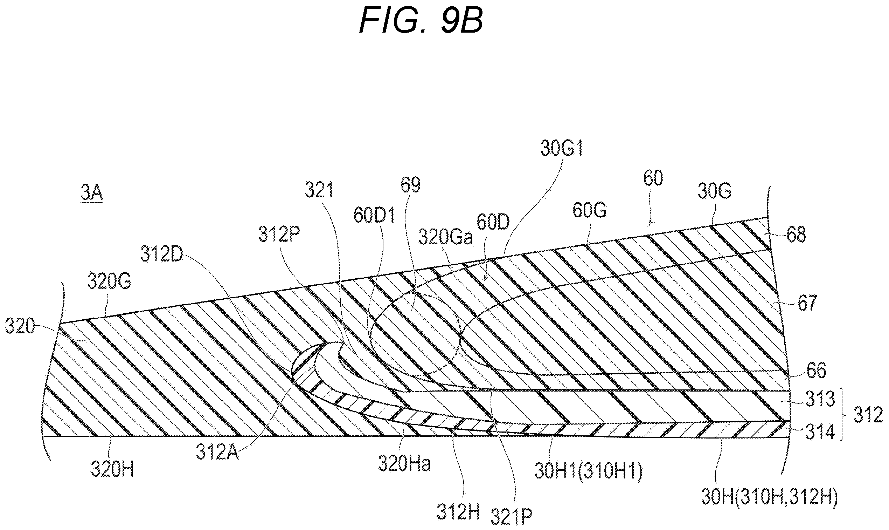

A modification of the distal portion 3A of the balloon catheter 3 of the present embodiment will be described with reference to Fig. 9B. As illustrated in Fig. 9B, in the cross-sectional view in the axial direction, the distal portion 312A of the inner tube shaft 312 is formed to intrude in an arc outward in the radial direction (upward in the same figure) from the inner surface 320H of the distal member 320. In other words, in the cross-sectional view in the axial direction, the distal portion 312A of the inner tube shaft 312 extends to be curved outward in the radial direction and toward the distal side (left side in the same figure) of the balloon catheter 3 from a lumen 30H of the inner tube shaft 312 as a starting point to a most distal end 312D, and extends to be curved outward in the radial direction and toward the proximal side (right side in the same figure) of the balloon catheter 3 from the most distal end 312D. More specifically, the distal portion 312A of the inner tube shaft 312 extends to be curved outward in the radial direction and toward the distal side (left side in the same figure) of the balloon catheter 3 from a site 30H1 of the lumen 30H of the balloon catheter 3 as the starting point to the most distal end 312D. The distal portion 312A of the inner tube shaft 312 extends from the most distal end 312D toward an apex portion 312P while extending toward the proximal side (right side in the same figure) of the balloon catheter 3.

The distal member 320 includes an interposed portion 321 that is interposed between the distal portion 312A of the inner tube shaft 312 and a distal end 60D of the balloon 60. The interposed portion 321 is engaged with the distal portion 312A of the inner tube shaft 312. The apex portion 312P of the distal portion 312A of the inner tube shaft 312 extends toward the proximal direction in the axial direction. As a result, the interposed portion 321 of the distal member 320 is disposed to be interposed between the apex portion 312P of the distal portion 312A of the inner tube shaft 312, which extends like a claw, and the distal end 60D of the balloon 60. The interposed portion 321 of the distal member 320 strengthens the engagement of the distal member 320, the inner tube shaft 312, and the balloon 60. The interposed portion 321 is positioned between the outer layer 313 of the inner tube shaft 312 and an inner layer 66 of the balloon 60, has a thickness gradually decreasing toward the proximal side, and extends to a terminal end 321P.

An inner surface-side proximal portion 320Ha of the distal member 320 is positioned in a section interposed between the inner surface 320H of the distal member 320 and an inner surface 312H of the inner tube shaft 312 in contact with the distal member 320. The thickness of the inner surface-side proximal portion 320Ha in the section increases along a curvature of the inner surface 312H from the site 310H1 of the lumen 310H of the inner tube shaft 312 toward the distal direction. An outer surface-side proximal portion 320Ga of the distal member 320 is positioned in a section interposed between an outer surface 320G of the distal member 320 and an outer surface 60G of the balloon 60. The thickness of the outer surface-side proximal portion 320Ga in the section increases along a curvature of the balloon 60 from a site 30G1 of an outer periphery 30G of the balloon catheter 3 toward the distal direction. The outer surface 320G of the distal member 320 and the outer surface 60G of the balloon 60 form a straight portion without a level difference in the cross-sectional view in the axial direction. The inner surface 320H of the distal member 320 and the inner surface 312H of the inner layer 314 of the inner tube shaft 312 facing the lumen 310H form a straight portion without a level difference in the cross-sectional view in the axial direction. The straight portion formed by the outer surface 320G of the distal member 320 and the outer surface 60G of the balloon 60 is inclined to intersect with respect to the straight portion of the inner surface 312H so as to form an intersection point by extension lines of respective straight portions on the distal side of the balloon catheter 3. The site 30G1 of the outer surface-side proximal portion 320Ga of the distal member 320 is positioned on the distal side in the axial direction with respect to the site 30H1 of the inner surface-side proximal portion 320Ha. The terminal end 321P of the interposed portion 321 is positioned closer to the distal side in the axial direction than the site 30G1 of the outer surface-side proximal portion 320Ga of the distal member 320. The terminal end 321P of the interposed portion 321 is positioned closer to the distal side in the axial direction than the site 30H1 of the inner surface-side proximal portion 320Ha of the distal member 320.

As illustrated in Fig. 8, the inner tube shaft 312 is provided with two X-ray radiopaque markers 270 indicating the position of the balloon 60 in the axial direction. As the X-ray radiopaque markers 270, for example, a thin metal wire formed of an X-ray radiopaque material, such as a metal such as platinum, gold, silver, iridium, titanium, or tungsten, or an alloy thereof can be used. As the X-ray radiopaque markers 270, a resin material containing powder made of an X-ray radiopaque material may be used.

As illustrated in Fig. 8, the proximal portion 65 of the balloon 60 is bonded to the distal portion 311A of the outer tube shaft 311. As illustrated in Fig. 9A, a distal portion 60A of the balloon 60 is formed to intrude in an arc inward in the radial direction from the outer surface of the distal member 320. In other words, as illustrated in Figs. 9A and 9B, the distal portion 60A of the balloon 60 is formed in a convex shape to be narrowed toward the distal side. The distal end 60D of the distal portion 60A of the balloon 60 is rounded. In a case where a perpendicular line is drawn along the axial direction so as to be brought into contact with the most distal end 60D1 of the distal portion 60A of the balloon 60, the distal member 320, the inner layer 314, the outer layer 313, the distal member 320, the most distal end 60D1 of the distal portion 60A of the balloon 60, and the distal member 320 are positioned in this order in the radial direction from the center axis.

In the cross-sectional view in the axial direction, the distal portion 60A of the balloon 60 includes a wedge portion 60A1 that is wedge-shaped as illustrated in Fig. 9A. The wedge portion 60A1 includes the parallel portion 60A2 parallel to the axial direction. According to this configuration, since the rigidity gradually transitions from the distal side to the proximal side of the balloon 60, bending at a portion where the balloon 60 is fused to the inner tube shaft 312 is smooth.

As illustrated in Fig. 8, the balloon catheter 3 includes an outer diameter constant portion 3E, a first tapered portion 3F, a second tapered portion 3G, and a third tapered portion 3H in order from the distal side. The inclination of the second tapered portion 3G with respect to the axial direction is larger than the inclination of the first tapered portion 3F. The inclination of the third tapered portion 3H with respect to the axial direction is smaller than the inclination of the second tapered portion 3G. The above-described multi-stage tapered portion facilitates insertion into and passing through a stenosis. Each of the tapered portions described above is formed under the pressure from the direction corresponding to the inclination. The third tapered portion 3H may be an outer diameter constant portion having a substantially constant outer diameter.

As illustrated in Fig. 9A, a thickness D1 at the distal end 60D of the balloon 60 positioned at the portion fused to the inner tube shaft 312 is equal to or smaller than a thickness D2 of the inner tube shaft 312 at a site P closer to the proximal side than the portion where the balloon 60 is bonded to the inner tube shaft 312, and is larger than a thickness D3 of the inner tube shaft 312 at the portion bonded to the balloon 60. According to this configuration, since the balloon 60 is inserted into the inner tube shaft 312, the thickness of the balloon 60 is incorporated, and an increase in rigidity can be suppressed. That is, the flexibility in the distal portion 3A of the balloon catheter 3 can be maintained.

As illustrated in Fig. 9A, the balloon 60 includes the inner layer 66, the base layer 67, and the outer layer 68 in order from the inner side. At the distal end 60D of the balloon 60, the inner layer 66 and the outer layer 68 are mixed with each other to form the mixed layer 69.

Next, materials of the inner layer, the outer layer, and the base layer which are components of the balloon will be described.

The base layer preferably contains a polyamide. In the base layer containing a polyamide, the polyamide accounts for preferably 50% by weight or more, more preferably 80% by weight or more, most preferably 100% by weight (that is, consisting of a polyamide) in a resin constituting the base layer. In a case where polyamide is contained as the resin of the base layer, pressure resistance strength and low compliance characteristics required of the catheter balloon can be ensured, which is preferable. The base layer may contain a known additive or a contrast medium used for, for example, X-ray as necessary, or may be composed only of a polyamide.

As the polyamide, those described in the section of the above-described catheter shaft can be appropriately selected and used.

The polyamide resin can be used singly or in combination of two or more types thereof.

Examples of the additive contained in the base layer as necessary include higher alcohols, hydroxybenzoic acid esters, and aromatic sulfonamides, but are not necessarily limited thereto.

In the cross section perpendicular to the axis at the axial center portion of the balloon, the cross-sectional area ratio of the base layer is preferably 30% to 70%, and more preferably 35% to 60%.

The average thickness of the base layer is preferably 5 to 20 µm. The thickness of the base layer may be set to an appropriate thickness from the viewpoint of a balloon diameter, required rupture resistance performance, and passability.

The average thickness of the base layer in a membranous main body constituting the balloon is calculated by conversion with an original tube design dimension and the thickness of the balloon.

The outer layer of the balloon (hereinafter, also referred to as a balloon outer layer) preferably contains an elastomer. Moreover, the inner layer of the balloon (hereinafter, also referred to as a balloon inner layer) and the balloon outer layer preferably contain an elastomer. The elastomer contained in the balloon outer layer preferably includes a polyamide elastomer. In the outer layer containing a polyamide elastomer, the polyamide elastomer accounts for preferably 50% by weight or more, more preferably 80% by weight or more, most preferably 100% by weight (that is, consisting of a polyamide elastomer resin) in a resin constituting the outer layer. In a case where the balloon is attached to the catheter and inserted into a body, with an outer layer containing an elastomer (preferably the polyamide elastomer) formed on the outermost layer, the balloon is flexible and thus excellent in passability through a blood vessel or a lumen in a living body.

The average thickness of the membranous main body constituting the balloon is preferably 5 to 15 µm, and more preferably 5 to 10 µm. In a case where the average thickness is within a range of 5 to 15 µm, abrasion resistance against a hard component such as a calcified lesion can be improved, and the procedure can be applied more safely.

In the cross section perpendicular to the axis at the axial center portion of the balloon, the cross-sectional area ratio of the outer layer is preferably 20% to 50%, and more preferably 25% to 50%.

Within this range, both rupture resistance performance and expansion performance of a hard lesion such as a calcified lesion can be achieved.

The balloon inner layer preferably contains a polyamide elastomer because of good adhesiveness with the base layer and resistance to delamination. Furthermore, in the inner layer containing a polyamide elastomer, the polyamide elastomer accounts for preferably 50% by weight or more, more preferably 80% by weight or more, most preferably 100% by weight (that is, consisting of a polyamide elastomer resin) in a resin constituting the inner layer.

In a case where the inner layer containing a polyamide elastomer is formed on the innermost side, it is possible to provide a catheter balloon exhibiting stable pressure resistance and to impart flexibility to the entire balloon, resulting in ensuring excellent passability in a blood vessel or a body cavity.

In the case where the inner layer containing a polyamide elastomer is formed on the innermost side and the outer layer 313 of the inner tube shaft 312 contains a polyamide elastomer, affinity between both is enhanced, and the bonding strength is improved, which are preferable.

The average thickness of the balloon inner layer is preferably 0.1 to 10 µm, and more preferably 0.1 to 7 µm. In a case where the average thickness is within a range of 0.1 to 10 µm, the flexibility of the entire balloon and low compliance characteristics are balanced, which is preferable.

As the polyamide elastomer contained in the inner layer and outer layer of the balloon, the same materials as the polyamide elastomer in the section of the above-described catheter shaft can be used. Thus, the description of the polyamide elastomer will not be repeated herein.

As described above, a preferable aspect of the balloon preferably includes a membranous main body provided with an inner layer that contains a polyamide elastomer and is provided on the innermost side, a base layer that contains a polyamide and is laminated on a surface of the inner layer, and an outer layer that contains a polyamide elastomer and is further provided on the outer side of the base layer. As a result, it is possible to provide the balloon exhibiting the excellent low compliance characteristics without impairing the balance between pressure resistance performance and passability performance.

The balloon catheter 3 according to the third embodiment can be manufactured by passing the bonded body including the distal member and catheter shaft and the balloon produced in the first embodiment described above through the core material, and employing the same steps as those in the manufacturing method in the first embodiment described above.

As described above, the balloon catheter 3 according to the third embodiment includes the shaft 310 that extends in the axial direction, the distal member 320 that is fused to the distal side of the shaft 310 and is more flexible than the shaft 310, and the balloon 60 disposed on the outer periphery of the shaft 310. The shaft 310 includes the outer layer 313 and the inner layer 314 disposed inward in the radial direction of the outer layer 313. In the cross-sectional view in the axial direction, the distal portion 312A of the inner tube shaft 312 intrudes in an arc outward in the radial direction from the inner surface of the distal member 320, and the distal portion 60A of the balloon 60 intrudes in an arc inward in the radial direction from the outer surface of the distal member 320. According to the balloon catheter 3 configured in such a manner, the contact area between the inner layer 314 and the distal member 320 can be increased, and a sufficient bonding strength between the shaft 310 and the distal member 320 can be obtained even though the fusion length is short. Therefore, it is possible to provide the balloon catheter 3 in which the bonding strength between the shaft 310 and the distal member 320 is increased while the flexibility of the distal portion 3A of the balloon catheter 3 is maintained.

Furthermore, the thickness D1 at the distal end 60D of the balloon 60 positioned at the portion fused to the inner tube shaft 312 is equal to or smaller than a thickness D2 of the inner tube shaft 312 at a site P closer to the proximal side than the portion where the balloon 60 is bonded to the inner tube shaft 312, and is larger than a thickness D3 of the inner tube shaft 312 at the portion bonded to the balloon 60. According to this configuration, since the balloon 60 is inserted into the inner tube shaft 312, the thickness of the balloon 60 is incorporated, and an increase in rigidity can be suppressed. That is, the flexibility in the distal portion 3A of the balloon catheter 3 can be maintained.

Furthermore, in the cross-sectional view in the axial direction, the distal portion 60A of the balloon 60 includes a wedge portion 60A1 that is wedge-shaped, and the wedge portion 60A1 includes a parallel portion 60A2 parallel to the axial direction. According to the balloon catheter 3 configured in such a manner, since the rigidity gradually transitions from the distal side to the proximal side of the balloon 60, bending at a portion where the balloon 60 is fused to the inner tube shaft 312 is smooth.

Furthermore, the crystallinity of the inner layer 314 of the inner tube shaft 312 decreases from the proximal side toward the distal side. According to the balloon catheter 3 configured in such a manner, the bending strength can be improved while the flexibility is maintained in the vicinity of the fusion portion on the distal member 20 side.

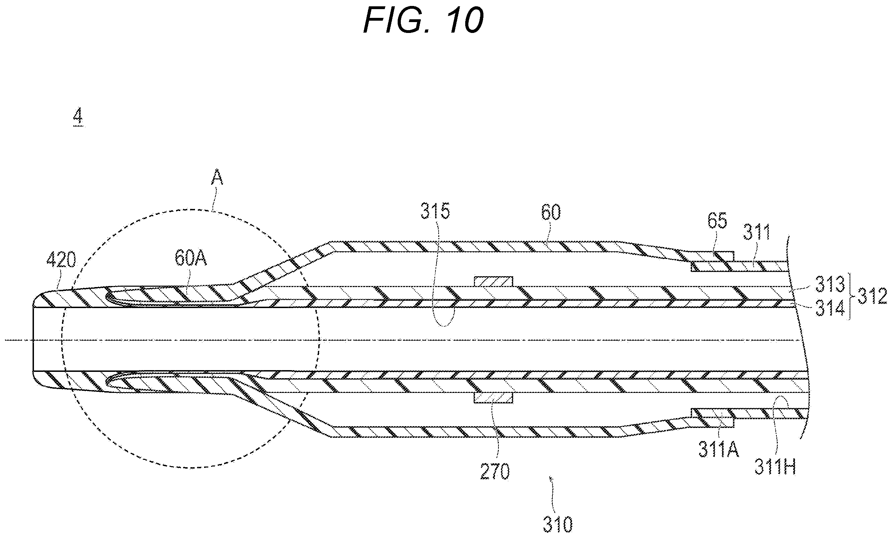

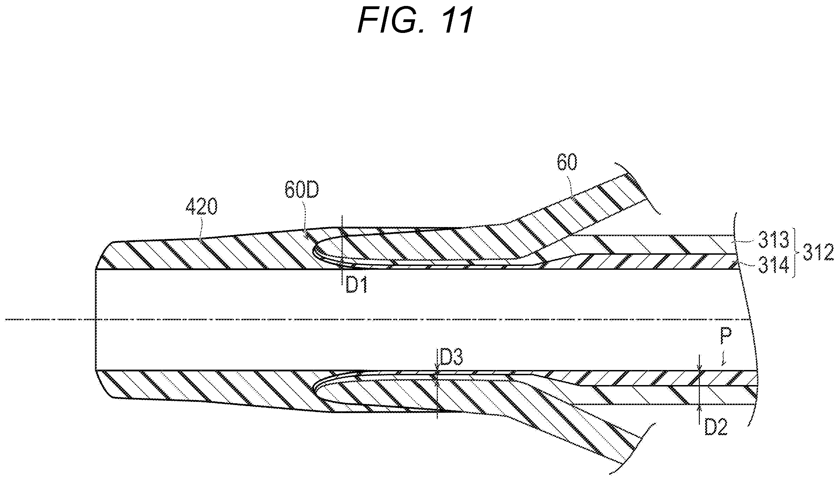

Next, a configuration of a balloon catheter 4 according to a modification of the third embodiment will be described with reference to Figs. 10 and 11. Fig. 10 is a diagram of a balloon catheter 4 according to the modification of the third embodiment, which corresponds to Fig. 8. Fig. 11 is a diagram of the balloon catheter 4 according to the modification of the third embodiment, which corresponds to Fig. 9A. The balloon catheter 4 according to the modification of the third embodiment is different from the balloon catheter 3 according to the third embodiment in the configuration of a distal member 420 and the like.

As illustrated in Figs. 10 and 11, the distal member 420 is configured such that the distal portion 60A of the balloon 60 more deeply intrudes outward in the radial direction as compared with the distal member 320 of the balloon catheter 3 according to the third embodiment. According to this configuration, detachment between the balloon 60 and the distal member 420 can be suitably prevented.

Furthermore, in the balloon catheter 4 according to the modification, as illustrated in Fig. 11, the thickness D1 at the distal end 60D of the balloon 60 positioned at the portion fused to the inner tube shaft 312 is equal to or smaller than a thickness D2 of the inner tube shaft 312 at a site P closer to the proximal side than the portion where the balloon 60 is bonded to the inner tube shaft 312, and is larger than a thickness D3 of the inner tube shaft 312 at the portion bonded to the balloon 60. According to this configuration, since the balloon 60 is inserted into the inner tube shaft 312, the thickness of the balloon 60 is incorporated, and an increase in rigidity can be suppressed. That is, the flexibility in the distal portion 3A of the balloon catheter 3 can be maintained.

### <Fourth Embodiment>

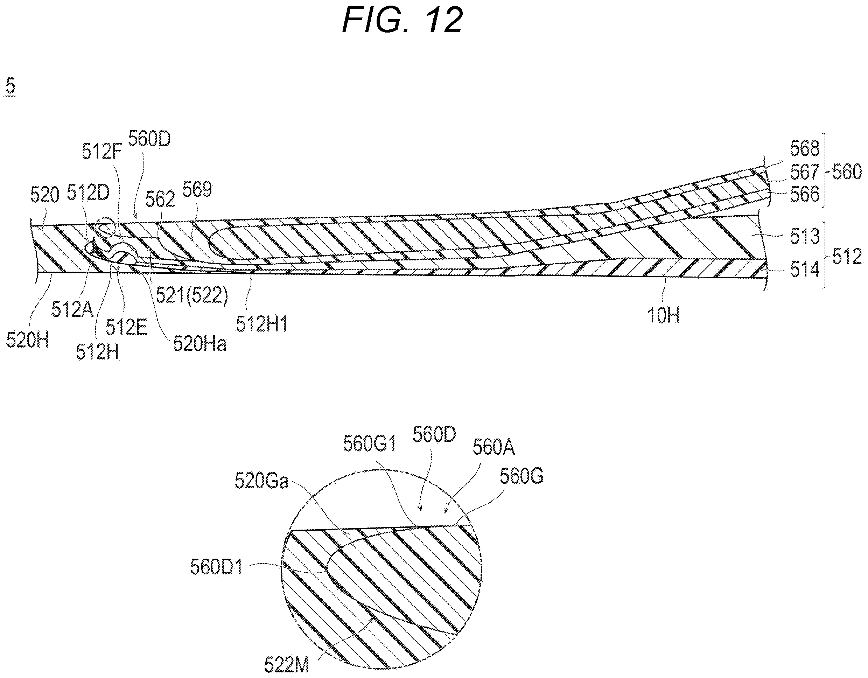

Next, a balloon catheter 5 according to a fourth embodiment of the present invention will be described with reference to Fig. 12. Fig. 12 is a diagram of the balloon catheter 5 according to the fourth embodiment, which corresponds to Fig. 9B.

As illustrated in Fig. 12, the balloon catheter 5 according to the fourth embodiment includes an inner tube shaft 512, a distal member 520 disposed on the distal side of the inner tube shaft 512, and a balloon 560 disposed on the outer periphery of the inner tube shaft 512.

As illustrated in Fig. 12, the inner tube shaft 512 includes an outer layer 513 and an inner layer 514 disposed inward in the radial direction of the outer layer 513.

In the cross-sectional view in the axial direction, as illustrated in Fig. 12, a distal portion 512A of the inner tube shaft 512 is formed to intrude in an arc outward in the radial direction from an inner surface 520H of the distal member 520. More specifically, the distal portion 512A of the inner tube shaft 512 extends to be curved outward in the radial direction and toward the distal side of the balloon catheter 5 from a site 512H1 of a lumen 512H of the inner tube shaft 512 as the starting point to a most distal end 512D.

At a site 512E positioned on the proximal side from the distal portion 512A of the inner tube shaft 512, the inner tube shaft 512 is configured to inflate outward in the radial direction. According to this configuration, since an inflating site 512F is in such a positional relationship as to be engaged with an interposed portion 521 of the distal member 520 described later, the bonding strength of the inner tube shaft 512 to the distal member 520 is improved.

The distal member 520 includes an interposed portion 521 that is interposed between the distal portion 512A of the inner tube shaft 512 and a distal end 560D of the balloon 560. In other words, the proximal portion 522 of the distal member 520 is positioned between the distal end 560D of the balloon 560 and the distal portion 512A of the inner tube shaft 512. According to this configuration, even though the distal member 520 is bent, the interposed portion 521 functions as a cushioning material between the distal end 560D of the balloon 560 and the distal portion 512A of the inner tube shaft 512. Thus, it is possible to minimize curling or detachment of the distal end 560D of the balloon 560. It is considered that the interposed portion 521 is formed because the distal member 520 flows between the inner tube shaft 512 and the balloon 560 during molding.

An inner surface-side proximal portion 520Ha of the distal member 520 is positioned in a section interposed between the inner surface 520H of the distal member 520 and an inner surface 512H of the inner tube shaft 512 in contact with the distal member 520. The thickness of the inner surface-side proximal portion 520Ha in the section increases along a curvature of the inner surface 512H from the site 510H1 of the lumen 510H of the inner tube shaft 512 toward the distal direction. The distal portion 512A of the inner tube shaft 512 is positioned between the inner surface-side proximal portion 520Ha of the distal member 520 and the proximal portion 522 (interposed portion 521) of the distal member 520. The distal portion 512A, the inner surface-side proximal portion 520Ha, and the interposed portion 521 are in contact with each other by fusion.

As illustrated in Fig. 12, the distal member 520 is disposed to wrap the distal end 560D of the balloon 560. The distal member 520 covers the radially outer side of a most distal end 560D1 of the balloon 560 with an outer surface-side proximal portion 520Ga of the distal member 520, and a curved boundary is configured with the outer surface-side proximal portion 520Ga and from the most distal end 560D1 to a site 560G1 of the outer periphery 560G. The distal member 520 covers the radially inner side of the most distal end 560D1 with a middle proximal portion 522M of the distal member 520, and defines a curved boundary with the middle proximal portion 522M from the most distal end 560D1 toward the proximal side. In other words, the most distal end 560D1 of the distal end 560D of the balloon 560 is covered with the outer surface-side proximal portion 520Ga and the middle proximal portion 522M of the distal member 520. According to this configuration, a bonded area between the distal member 520 and the distal portion 560A of the balloon 560 is increased, and the curling of the distal end 560D of the balloon 560 is minimized, so that the bonding strength is improved.

As illustrated in Fig. 12, the distal portion 560A of the balloon 560 is formed to intrude in an arc inward in the radial direction from the outer surface of the distal member 520.

As illustrated in Fig. 12, the distal end 560D of the balloon 560 is formed in a tongue shape toward the distal side of the balloon catheter 5. The distal end 560D of the balloon 560 is configured to have a thickness decreasing toward the distal side of the site 562, and is configured to have a thickness increasing toward the proximal side of the site 562.

The balloon 560 includes an inner layer 566, a base layer 567, and an outer layer 568 similarly to the balloon 60 according to the third embodiment described above. At the distal portion 560A of the balloon 560, the inner layer 566 and the outer layer 568 are mixed with each other to form a mixed layer 569.

### <Fifth Embodiment>

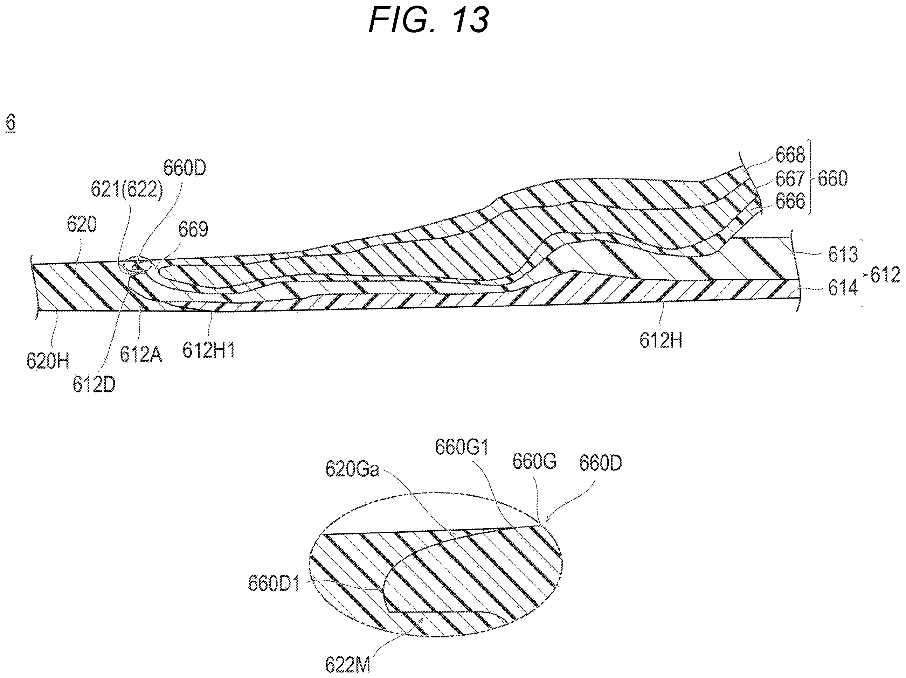

Next, a balloon catheter 6 according to a fifth embodiment of the present invention will be described with reference to Fig. 13. Fig. 13 is a diagram of the balloon catheter 6 according to the fifth embodiment, which corresponds to Fig. 9B.

As illustrated in Fig. 13, the balloon catheter 6 according to the fifth embodiment includes an inner tube shaft 612, a distal member 620 disposed on the distal side of the inner tube shaft 612, and a balloon 660 disposed on the outer periphery of the inner tube shaft 612.

As illustrated in Fig. 13, the inner tube shaft 612 includes an outer layer 613 and an inner layer 614 disposed inward in the radial direction of the outer layer 613.

In the cross-sectional view in the axial direction, as illustrated in Fig. 13, a distal portion 612A of the inner tube shaft 612 is formed to intrude in an arc outward in the radial direction from an inner surface 620H of the distal member 620. More specifically, the distal portion 612A of the inner tube shaft 612 extends to be curved outward in the radial direction and toward the distal side of the balloon catheter 6 from a site 612H1 of a lumen 612H of the inner tube shaft 612 as the starting point to a most distal end 612D.

The distal member 620 includes an interposed portion 621 that is interposed between the distal portion 612A of the inner tube shaft 612 and a distal end 660D of the balloon 660. In other words, the proximal portion 622 of the distal member 620 is positioned between the distal end 660D of the balloon 660 and the distal portion 612A of the inner tube shaft 612. According to this configuration, even though the distal member 620 is bent, the interposed portion 621 functions as a cushioning material between the distal end 660D of the balloon 660 and the distal portion 612A of the inner tube shaft 612. Thus, it is possible to minimize curling or detachment of the distal end 660D of the balloon 660. It is considered that the interposed portion 621 is formed because the distal member 620 flows between the inner tube shaft 612 and the balloon 660 during molding. The interposed portion 621 according to the fifth embodiment is formed in a shorter length in the axial direction than the interposed portion 521 according to the fourth embodiment.

As illustrated in Fig. 13, the distal member 620 is disposed to wrap the distal end 660D of the balloon 660. The distal member 620 covers the radially outer side of a most distal end 660D1 of the balloon 660 with an outer surface-side proximal portion 620Ga of the distal member 620, and a curved boundary is configured with the outer surface-side proximal portion 620Ga and from the most distal end 660D1 to a site 660G1 of the outer periphery 660G. The distal member 620 covers the radially inner side of the most distal end 660D1 with a middle proximal portion 622M of the distal member 620, and defines a curved boundary with the middle proximal portion 622M from the most distal end 660D1 toward the proximal side. In other words, the most distal end 660D1 of the distal end 660D of the balloon 660 is covered with the outer surface-side proximal portion 620Ga and the middle proximal portion 622M of the distal member 620. According to this configuration, since the curling of the distal end 660D of the balloon 660 is minimized, the bonding strength is improved. Furthermore, according to this configuration, the bonded area between the distal member 620 and the distal end 660D of the balloon 660 is increased, and the bonding strength is improved.

As illustrated in Fig. 13, the distal portion 660A of the balloon 660 is formed to intrude in an arc inward in the radial direction from the outer surface of the distal member 620. As illustrated in Fig. 13, the distal end 660D of the balloon 660 is formed in a tongue shape toward the distal side of the balloon catheter 6.

The balloon 660 includes an inner layer 666, a base layer 667, and an outer layer 668 similarly to the balloon 60 according to the third embodiment described above. At the distal portion 661 of the balloon 660, the inner layer 666 and the outer layer 668 are mixed with each other to form a mixed layer 669.

### <Sixth Embodiment>

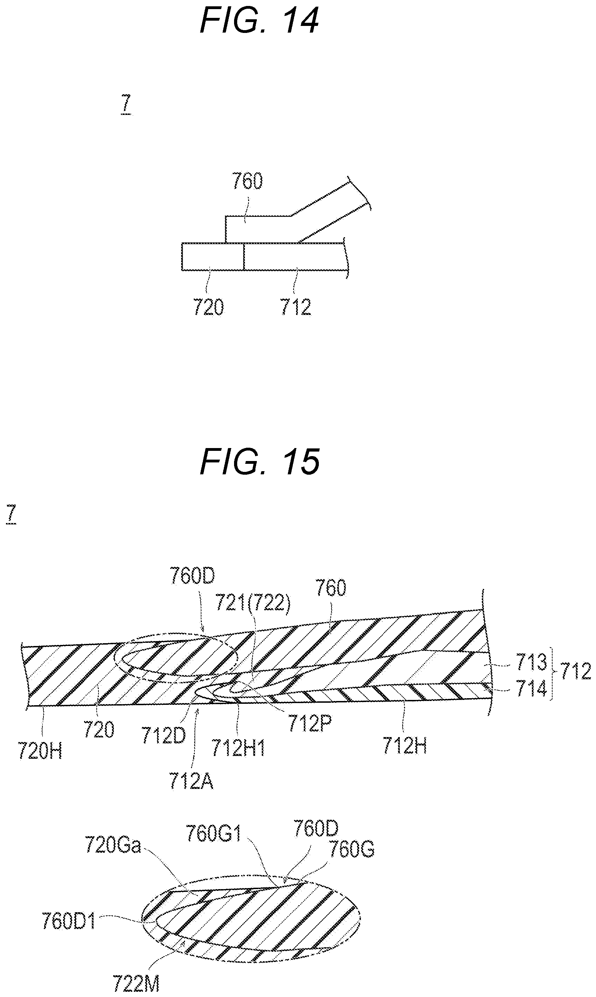

Next, a balloon catheter 7 according to a sixth embodiment of the present invention will be described with reference to Figs. 14 and 15. Fig. 14 is an image diagram illustrating a state before molding of the balloon catheter 7 according to the sixth embodiment. Fig. 15 is a diagram of the balloon catheter 7 according to the sixth embodiment, which corresponds to Fig. 9B.

As illustrated in Fig. 15, the balloon catheter 7 according to the sixth embodiment includes an inner tube shaft 712, a distal member 720 disposed on the distal side of the inner tube shaft 712, and a balloon 760 disposed on the outer periphery of the inner tube shaft 712. As illustrated in Fig. 14, in the balloon catheter 7 before molding, each member is disposed such that the distal member 720 and the balloon 760 have a relatively large overlapping length (for example, 0.2 mm).

As illustrated in Fig. 15, the inner tube shaft 712 includes an outer layer 713 and an inner layer 714 disposed inward in the radial direction of the outer layer 713.

In the cross-sectional view in the axial direction, as illustrated in Fig. 15, a distal portion 712A of the inner tube shaft 712 is formed to intrude in an arc outward in the radial direction from an inner surface 720H of the distal member 720. More specifically, the distal portion 712A of the inner tube shaft 712 extends to be curved outward in the radial direction and toward the distal side of the balloon catheter 7 from a site 712H1 of a lumen 712H of the inner tube shaft 712 as the starting point to a most distal end 712D, followed by extending to a terminal end 712P toward the proximal side.

The distal member 720 includes an interposed portion 721 that is interposed between the distal portion 712A of the inner tube shaft 712 and a distal end 760D of the balloon 760. In other words, the proximal portion 722 of the distal member 720 is positioned between the distal end 760D of the balloon 760 and the distal portion 712A of the inner tube shaft 712. According to this configuration, even though the distal member 720 is bent, the interposed portion 721 functions as a cushioning material between the distal end 760D of the balloon 760 and the distal portion 712A of the inner tube shaft 712. Thus, it is possible to minimize curling or detachment of the distal end 760D of the balloon 760. The reason why the interposed portion 721 is formed is because the distal member 720 flows between the inner tube shaft 712 and the balloon 760 during molding.

As illustrated in Fig. 15, the distal member 720 is disposed to wrap the distal end 760D of the balloon 760. The distal member 720 covers the radially outer side of a most distal end 760D1 of the balloon 760 with an outer surface-side proximal portion 720Ga of the distal member 720, and a curved boundary is configured with the outer surface-side proximal portion 720Ga and from the most distal end 760D1 to a site 760G1 of the outer periphery 760G. The distal member 720 covers the radially inner side of the most distal end 760D1 with a middle proximal portion 722M of the distal member 720, and defines a curved boundary with the middle proximal portion 722M from the most distal end 760D1 toward the proximal side. In other words, the most distal end 760D1 of the distal end 760D of the balloon 760 is covered with the outer surface-side proximal portion 720Ga and the middle proximal portion 722M of the distal member 720. According to this configuration, since the curling of the distal end 760D of the balloon 760 is minimized, the bonding strength is improved. According to this configuration, the bonded area between the distal member 720 and the distal portion 760D of the balloon 760 is increased, and the bonding strength is improved.

As illustrated in Fig. 15, the distal end 760D of the balloon 760 is formed to intrude in an arc inward in the radial direction from the outer surface of the distal member 720. As illustrated in Fig. 15, the distal end 760D of the balloon 760 is formed in a tongue shape toward the distal side of the balloon catheter 7. The position in the axial direction of the most distal end 760D1 of the balloon 760 is disposed closer to the distal side than the position in the axial direction of the most distal end 712D of the inner tube shaft 712.

The balloon 760 of the balloon catheter 7 according to the sixth embodiment is configured to include a single layer. Since the balloon 760 is composed of a single layer, the thermal ductility of the balloon 760 is increased, and the most distal end of the balloon 760 is positioned closer to the distal side than the most distal end of the inner tube shaft 712 as illustrated in Fig. 15.

The material constituting the balloon 760 composed of a single layer is not particularly limited, but for example, the above-described polyamide elastomer can be used.

### <Modification of Sixth Embodiment>

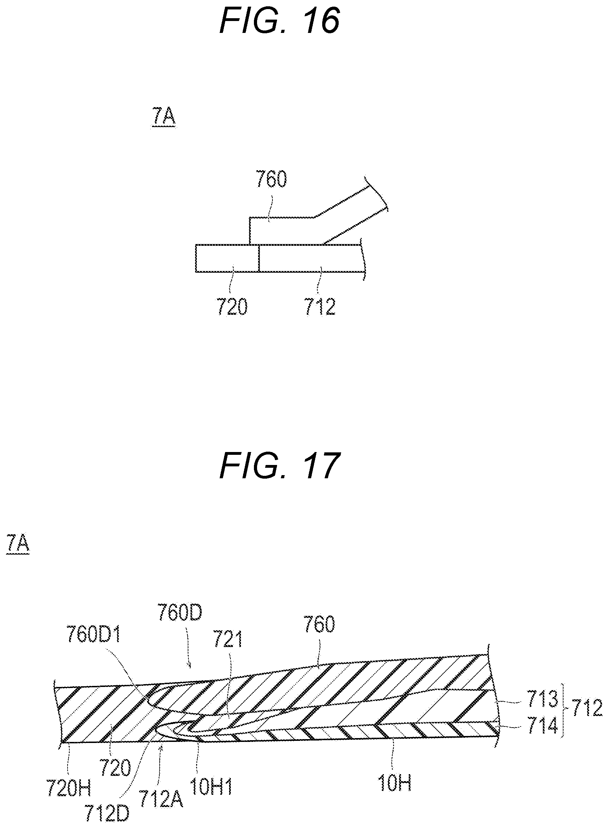

Next, a balloon catheter 7A according to a modification of the sixth embodiment of the present invention will be described with reference to Figs. 16 and 17. Fig. 16 is an image diagram illustrating a state before molding of the balloon catheter 7A according to the modification of the sixth embodiment. Fig. 17 is a diagram of the balloon catheter 7A according to the sixth embodiment, which corresponds to Fig. 9B.

As illustrated in Fig. 17, the balloon catheter 7A according to the modification of the sixth embodiment includes the inner tube shaft 712, the distal member 720 disposed on the distal side of the inner tube shaft 712, and the balloon 760 disposed on the outer periphery of the inner tube shaft 712. As illustrated in Fig. 16, in the balloon catheter 7A before molding, each member is disposed such that the distal member 720 and the balloon 760 have a relatively small overlapping length (for example, 0.1 mm).

In the balloon catheter 7A according to the modification of the sixth embodiment, since the respective members are disposed so that the distal member 720 and the balloon 760 have a relatively small overlapping length (for example, 0.1 mm), and molding is then performed, the inner tube shaft 712 is position closer to the distal side as compared to the balloon catheter 7 according to the sixth embodiment, as illustrated in Fig. 17. The position in the axial direction of the most distal end 760D1 of the balloon 760 is disposed substantially the same as the position in the axial direction of the most distal end 712D of the inner tube shaft 712.

### <Seventh Embodiment>

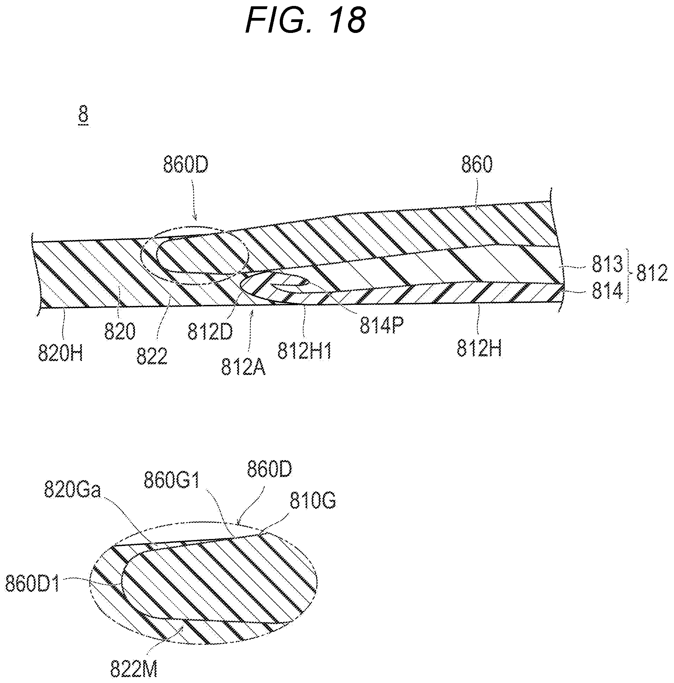

Next, a balloon catheter 8 according to a seventh embodiment of the present invention will be described with reference to Fig. 18. Fig. 18 is a diagram of the balloon catheter 8 according to the seventh embodiment, which corresponds to Fig. 9B.

As illustrated in Fig. 18, the balloon catheter 8 according to the seventh embodiment includes an inner tube shaft 812, a distal member 820 disposed on the distal side of the inner tube shaft 812, and a balloon 860 disposed on the outer periphery of the inner tube shaft 812.

As illustrated in Fig. 18, the inner tube shaft 812 includes an outer layer 813 and an inner layer 814 disposed inward in the radial direction of the outer layer 813.

In the cross-sectional view in the axial direction, as illustrated in Fig. 18, a distal portion 812A of the inner tube shaft 812 is formed to intrude in an arc outward in the radial direction from an inner surface 820A of the distal member 820. More specifically, the distal portion 812A of the inner tube shaft 812 extends to be curved outward in the radial direction and toward the distal side of the balloon catheter 8 from a site 812H1 of a lumen 812H of the inner tube shaft 812 as the starting point to a most distal end 812D.

As illustrated in Fig. 18, the inner layer 814 is configured to be bent to extend toward the distal end, followed by extending toward the proximal end. The inner layer 814 extends from the most distal end 812D of the inner tube shaft 812 toward the proximal side, and terminates at a terminal end 814P in the middle of the outer layer 813 in the radial direction. The inner layer 814 is configured to be pressed and crumpled by the distal portion 861 of the balloon 860.

As illustrated in Fig. 18, the distal member 820 is disposed to wrap the distal end 860D of the balloon 860. The distal member 820 is disposed to wrap the distal end 860D of the balloon 860. The distal member 820 covers the radially outer side of a most distal end 860D1 of the balloon 860 with an outer surface-side proximal portion 820Ga of the distal member 820, and a curved boundary is configured with the outer surface-side proximal portion 820Ga and from the most distal end 860D1 to a site 860G1 of the outer periphery 860G. The distal member 820 covers the radially inner side of the most distal end 860D1 with a middle proximal portion 822M of the distal member 820, and defines a curved boundary with the middle proximal portion 822M from the most distal end 860D1 toward the proximal side. In other words, the most distal end 860D1 of the distal end 860D of the balloon 860 is covered with the outer surface-side proximal portion 820Ga and the middle proximal portion 822M of the distal member 820. According to this configuration, since the curling of the distal end 860D of the balloon 860 is minimized, the bonding strength is improved. According to this configuration, the bonded area between the distal member 820 and the distal portion 861 of the balloon 860 is increased, and the bonding strength is improved.

As illustrated in Fig. 18, the distal end 860D of the balloon 860 is formed to intrude in an arc inward in the radial direction from the outer surface of the distal member 820. As illustrated in Fig. 1, the distal end 860D of the balloon 860 is formed in a tongue shape toward the distal side of the balloon catheter.

The balloon 860 of the balloon catheter 8 according to the seventh embodiment is configured to include a single layer. Since the balloon 860 is composed of a single layer, the thermal ductility of the balloon 860 is increased, and the most distal end of the balloon 860 is positioned closer to the distal side than the most distal end of the inner tube shaft 812 as illustrated in Fig. 18.

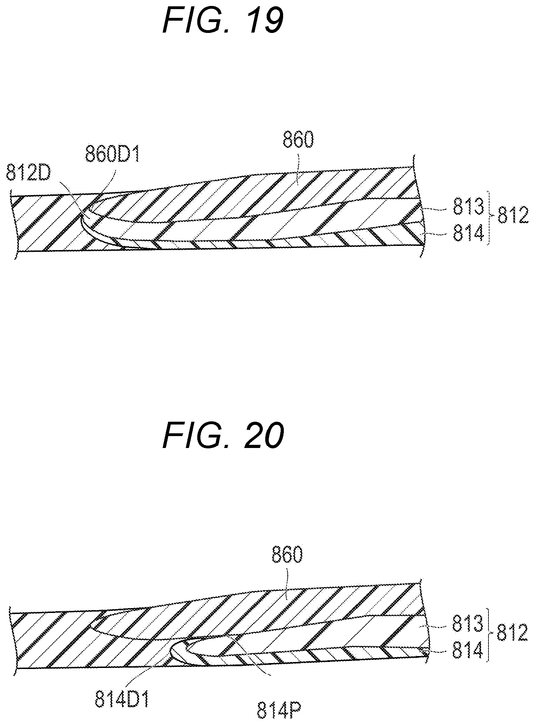

As illustrated in Fig. 19, as compared with the balloon catheter 8 according to the seventh embodiment, the inner tube shaft 812 is disposed on the distal side, and the balloon 860 and the inner tube shaft 812 are fixed without a gap. According to this configuration, the bonding strength between the balloon 860 and the inner tube shaft 812 is improved. The distal end 812D of the inner tube shaft 812 is provided at the position to cover the most distal end 860D1 of the balloon 860. According to this configuration, since the curling of the distal end 860D of the balloon 860 is minimized, the bonding strength is improved.

In the balloon catheter 8 according to the seventh embodiment, the inner layer 814 is configured to be pressed and crumpled by the distal portion 861 of the balloon 860, but may be configured not to be pressed and crumpled as illustrated in Fig. 20. That is, the inner layer 814 extends from the most distal end 814D1 of the inner tube shaft 812 toward the proximal side while gradually decreasing in thickness, extends between the outer layer 813 and the balloon 860, and terminates at an apex portion 814P.

### <Eighth Embodiment>

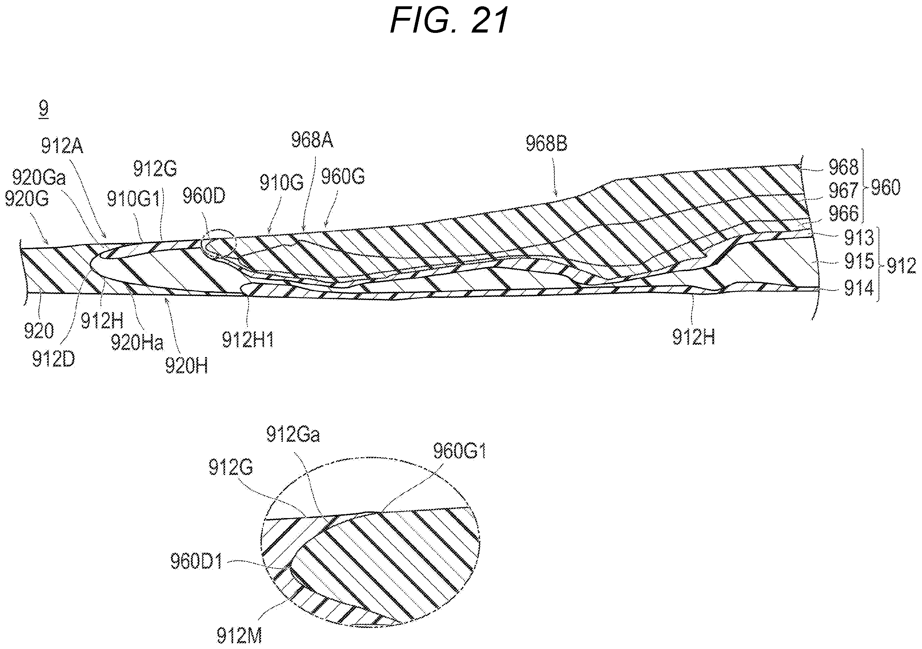

Next, a balloon catheter 9 according to an eighth embodiment will be described with reference to Fig. 21. Fig. 21 is a diagram of the balloon catheter 9 according to the eighth embodiment, which corresponds to Fig. 9B.

As illustrated in Fig. 21, the balloon catheter 9 according to the eighth embodiment includes an inner tube shaft 912, a distal member 920 disposed on the distal side of the inner tube shaft 912, and a balloon 960 disposed on the outer periphery of the inner tube shaft 912.

As illustrated in Fig. 21, the inner tube shaft 912 includes an outer layer 913, an inner layer 914 disposed inward in the radial direction of the outer layer 913, and a middle layer 915 disposed between the outer layer 913 and the inner layer 914. As a material constituting the middle layer 915, the same material as the material constituting the outer layer 913 can be used.

In the cross-sectional view in the axial direction, as illustrated in Fig. 21, a distal portion 912A of the inner tube shaft 912 is formed to intrude in an arc outward in the radial direction from an inner surface 920H of the distal member 920. More specifically, the distal portion 912A of the inner tube shaft 912 extends to be curved outward in the radial direction and toward the distal side of the balloon catheter 9 from a site 912H1 of a lumen 912H of the inner tube shaft 912 as the starting point to a most distal end 912D.

An outer surface 912G of the distal portion 912A of the shaft 912 constitutes an outer periphery 910G of the balloon catheter 9. The distal portion 912A of the inner tube shaft 910 is interposed between the distal member 920 and the most distal end 960D1 of the balloon 960 in the axial direction. In a case where a line parallel to the axial direction of the inner tube shaft 912 is drawn to pass through the most distal end 912D of the inner tube shaft 912, the distal member 920, the inner tube shaft 912, and the balloon 960 are disposed in this order from the distal end in the balloon catheter 9. The outer layer 913 and the middle layer 915 of the inner tube shaft 910 extend closer to the distal side than the inner layer 914.

As illustrated in Fig. 21, the distal member 920 is disposed to wrap around the distal portion 912A of the inner tube shaft 912. An inner surface-side proximal portion 920Ha of the distal member 920 is positioned in a section interposed between the inner surface 920H of the distal member 920 and an inner surface 912H of the inner tube shaft 912 in contact with the distal member 920. The thickness of the inner surface-side proximal portion 920Ha in the radial direction in the section increases along a curvature of the inner surface 912H from the site 912H1 of the lumen 912H of the inner tube shaft 912 toward the distal direction. An outer surface-side proximal portion 920Ga of the distal member 920 is positioned in a section interposed between the outer surface 920G of the distal member 920 and an outer surface 912G (forming a boundary with the distal member 920) of the inner tube shaft 912. The thickness of the outer surface-side proximal portion 920Ga in the radial direction in the section increases along a curvature extending from a site 910G1 of the outer surface 912G of the inner tube shaft 912 (the outer periphery 910G of the balloon catheter 9) and from the radially outer side toward the inner side of the inner tube shaft 912 in the distal direction. According to the configuration in which the distal member 920 is disposed to wrap the distal portion 912A of the inner tube shaft 912, the contact area between the inner tube shaft 912 and the distal member 920 increases, and the bonding strength between the inner tube shaft 912 and the distal member 920 is improved even though the fusion length is short. Furthermore, since the distal portion 912A of the inner tube shaft 912 is at the position interposed between the outer surface-side proximal portion 920Ga and the inner surface-side proximal portion 920Ha of the distal member 920, a strong bending force is dispersed by both proximal portions (920Ga and 920Ha) to maintain the connection even though the distal member 920 is inserted into a bent blood vessel, and the force is applied.

The most distal end 960D1 of the balloon 960 is positioned inward in the radial direction with respect to the outer surface 912G of the shaft 912. The shaft 912 covers the radially outer side of the most distal end 960D1 of the balloon 960 with the outer surface-side proximal portion 912Ga of the shaft 912, and a curved boundary is configured with the outer surface-side proximal portion 912Ga and from the most distal end 960D1 to a site 960G1 of the outer periphery 960G. The shaft 912 covers the radially inner side of the most distal end 960D1 with a middle portion 912M of the shaft 912, and defines a curved boundary at the portion of balloon 960 with the middle portion 912M from the most distal end 960D1 toward the proximal side. In other words, the most distal end 960D1 of the distal end 960D of the balloon 960 is covered with the outer surface-side proximal portion 912Ga and the middle portion 912M of the shaft 912. According to the configuration, since a most distal portion 961 of the balloon 960 is not disposed on the outer surface of the balloon catheter 9, curling can be reduced.

The balloon 960, the inner tube shaft 912, and the distal member 920 are configured such that there is no unevenness or level difference on the outer peripheral surface where they are continuous. According to this configuration, it is possible to suppress the balloon catheter 9 from being caught by a blood vessel or a stent. As illustrated in Fig. 1, the distal end 960D of the balloon 960 is formed in a tongue shape toward the distal side of the balloon catheter 9.

The balloon 960 includes an inner layer 966, a base layer 967, and an outer layer 968. As illustrated in Fig. 21, the outer layer 968 of the balloon 960 is configured such that the thickness at a proximal site 968B is thicker than that at a distal site 968A. According to this configuration, in a case where the outer layer 968 of the balloon 960 contains a polyamide elastomer, the proximal site 968B is easily bent.

### <Ninth Embodiment>

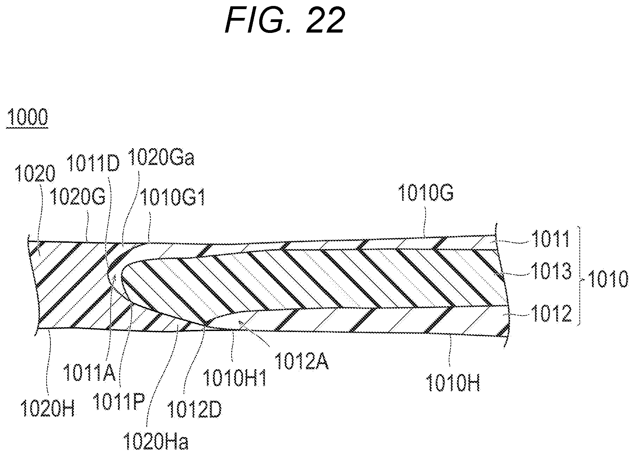

Next, a medical elongated body 1000 according to a ninth embodiment of the present invention will be described with reference to Fig. 22. Fig. 22 is a diagram of the medical elongated body 1000 according to the ninth embodiment, which corresponds to Fig. 3.

As illustrated in Fig. 22, the medical elongated body 1000 according to the ninth embodiment includes a catheter shaft 1010 that extends in the axial direction and a distal member 1020 that is disposed on the distal side of the catheter shaft 1010.

The catheter shaft 1010 includes an outer layer 1011, an inner layer 1012 that is positioned inward in the radial direction of the outer layer 1011, and a middle layer 1013 positioned between the outer layer 1011 and the inner layer 1012.

As illustrated in Fig. 22, in the cross-sectional view in the axial direction, a distal portion 1011A of the outer layer 1011 is formed to intrude in an arc inward in the radial direction (downward in Fig. 22) from an outer surface 1020G of the distal member 1020. In other words, in the cross-sectional view in the axial direction as illustrated in Fig. 22, the distal portion 1011A of the outer layer 1011 extends to be curved inward in the radial direction and toward the distal side (left side in Fig. 22) of the medical elongated body 1000 from a site 1010G1 of an outer surface 1010G of the catheter shaft 1010 as a starting point to the most distal end 1011D. The distal portion 1011A of the outer layer 1011 is positioned between the curved distal end of the middle layer 1013 and the curved proximal end of the distal member 1020 from the most distal end 1011D and terminates at an endpoint 1011P in the middle in the radial direction while gradually reducing the thickness.

A distal portion 1012A of the inner layer 1012 is formed to slightly intrude in an arc outward in the radial direction (upward in Fig. 22) from an inner surface 1020H of the distal member 1020. The distal portion 1012A of the inner layer 1012 extends inward in the radial direction and toward the distal side (left side in Fig. 22) of the medical elongated body 1000 from a site 1010H1 of an inner surface 1010H of the catheter shaft 1010 as the starting point to the most distal end 1012D.

As illustrated in Fig. 22, the distal member 1020 is disposed to wrap the distal portion 1011A of the outer layer 1011 of the catheter shaft 1010. An inner surface-side proximal portion 1020Ha of the distal member 1020 is positioned in a section interposed between the inner surface 1020H of the distal member 1020 and the inner surface 1010H of the catheter shaft 1010 in contact with the distal member 1020. The thickness of the inner surface-side proximal portion 1020Ha in the radial direction increases in the section along a curvature of the inner surface 1010H in the distal direction from the site 1010H1 of the lumen (inner surface) 1010H of the inner layer 1012 of the catheter shaft. An outer surface-side proximal portion 1020Ga of the distal member 1020 is positioned in a section interposed between the outer surface 1020G of the distal member 1020 and the outer surface 1010G of the catheter shaft 1010. The thickness of the outer surface-side proximal portion 1020Ga in the radial direction increases in the section along a curvature of the outer surface 1010G of the outer layer 1011 in the distal direction from the site 1010G1 of the outer surface 1010G of the catheter shaft 1010. According to this configuration, the contact area between the distal member 1020 and the catheter shaft 1010 is increased, and the bonding strength is improved.

A distal portion 1011A of the catheter shaft 1010 is formed in a tongue shape toward the distal side of the medical elongated body 1000. The distal member 1020 and the catheter shaft 1010 constitute a tongue-shaped boundary line. As a result, not only durability against bending but also durability against tension is provided.

In the present embodiment, the distal portion 1011A of the catheter shaft 1010 is formed in a tongue shape toward the distal side of the medical elongated body 1000, but a modification of the present embodiment can be made in which the distal portion 1011A of the catheter shaft 1010 is formed in a tongue shape toward the proximal side of the medical elongated body 1000.

As described above, the medical elongated body and the balloon catheter according to the present invention has been described through the embodiments, but the invention is not limited to the configurations described in the embodiment and is appropriately changed based on the claims.

For example, the medical elongated body described in the first embodiment may not be provided with the kink-resistant protector. Furthermore, the specific use of the medical elongated body is not particularly limited as long as the medical elongated body can be used for the purpose of introducing a medical device (a guide wire, various medical instruments for treatment, or the like) into a living body.

For example, the balloon catheter described in the third embodiment may be configured as a so-called over-the-wire type balloon catheter having a guide wire lumen extending from a distal end to a proximal end of the shaft.

Furthermore, the inner layer of the medical elongated body described in the first embodiment is configured such that the crystallinity decreases from the proximal side toward the distal side, but the present invention is not limited thereto.

In each embodiment including the above-described modification, a metal reinforcing body such as a blade or a coil may be provided between the outer layer and the inner layer of the catheter shaft or the inner tube shaft.

In each of the above-described embodiments, it has been described that the distal member is formed of a material softer than the shaft, but the modification of each embodiment includes a member formed of a material equal to or harder than the shaft.

The present application is based on Japanese Application No. 2022-156483 filed on September 29, 2022, the entire content of which is incorporated herein by reference.

### Reference Signs List

1, 2 Medical elongated body
1A, 2A Distal portion of medical elongated body
3 Balloon catheter
3A Distal portion of balloon catheter
10, 110 Catheter shaft
11, 111 Outer layer of catheter shaft
11A, 111A Distal portion of outer layer
12, 112 Inner layer of catheter shaft
12A, 112A Distal portion of inner layer
20, 120, 320 Distal member
20A Inner surface of distal member
20B Outer surface of distal member
60 Balloon
61A Wedge portion
61B Parallel portion
121 Interposed portion
310 Shaft
311 Outer tube shaft
311A Distal portion of outer tube shaft
312 Inner tube shaft
312A Distal portion of inner tube shaft
313 Outer layer of inner tube shaft
314 Inner layer of inner tube shaft

## Claims

1. A medical elongated body comprising:
a catheter shaft that extends in an axial direction and is provided with an outer layer and an inner layer that is disposed inward in a radial direction of the outer layer; and
a distal member that is fused to a distal side of the catheter shaft and is more flexible than the catheter shaft, wherein
in a cross-sectional view in the axial direction,
a distal portion of the inner layer intrudes in an arc outward in the radial direction from an inner surface of the distal member, and
a distal portion of the outer layer intrudes in an arc inward in the radial direction from an outer surface of the distal member.

2. A medical elongated body comprising:
a catheter shaft that extends in an axial direction and is provided with an outer layer and an inner layer that is disposed inward in a radial direction of the outer layer;
a distal member that is fused to a distal side of the catheter shaft and is more flexible than the catheter shaft, wherein
in a cross-sectional view in the axial direction,
a distal portion of the inner layer intrudes in an arc outward in the radial direction from an inner surface of the distal member, and
the distal member includes an interposed portion that is interposed between a distal portion of the inner layer and a distal portion of the outer layer.

3. A balloon catheter comprising:
a shaft that extends in an axial direction;
a distal member that is fused to a distal side of the shaft and is more flexible than the shaft; and
a balloon that is disposed on an outer periphery of the shaft, wherein
the shaft includes
an outer layer and
an inner layer that is disposed inward in a radial direction of the outer layer,
in a cross-sectional view in the axial direction,
a distal portion of the shaft intrudes in an arc outward in the radial direction from an inner surface of the distal member, and
a distal portion of the balloon intrudes in an arc inward in the radial direction from an outer surface of the distal member.

4. The balloon catheter according to claim 3, wherein
the shaft includes
an outer tube shaft that is provided with a lumen, and
an inner tube shaft that is disposed in the lumen of the outer tube shaft.

5. The balloon catheter according to claim 4, wherein a thickness at a distal end of the balloon positioned at a portion fused to the inner tube shaft is equal to or smaller than a thickness of the inner tube shaft at a site closer to the proximal side than a portion where the balloon is bonded to the inner tube shaft, and is larger than a thickness of the inner tube shaft at a portion bonded to the balloon.

6. The balloon catheter according to claim 3 or 4, wherein
in the cross-sectional view in the axial direction,
the distal portion of the balloon includes
a wedge portion that is wedge-shaped, and
the wedge portion includes a parallel portion parallel to the axial direction.

7. The balloon catheter according to claim 3 or 4, wherein in the cross-sectional view in the axial direction, an inner surface-side proximal portion is provided in a section interposed between the inner surface of the distal member and an inner surface of the shaft in contact with the distal member, at least a part of a proximal portion of the distal member is positioned between the distal portion of the balloon and the distal portion of the shaft, and the distal portion of the shaft is positioned between the at least a part of the proximal portion of the distal member and the inner surface-side proximal portion.

8. The balloon catheter according to claim 3 or 4, wherein in the cross-sectional view in the axial direction, the distal member is disposed to wrap around a distal end of the distal portion of the balloon.

9. The balloon catheter according to claim 3 or 4, wherein the distal member and the balloon contain a polyamide elastomer.

10. A medical elongated body comprising:
a catheter shaft that extends in an axial direction and is provided with an outer layer and an inner layer that is disposed inward in a radial direction of the outer layer; and
a distal member that is fused to a distal side of the catheter shaft, wherein
in a cross-sectional view in the axial direction,
a distal portion of the inner layer extends to be curved, along a distal portion of the outer layer, outward in a radial direction and toward a distal side from a site of a lumen of the catheter shaft as a starting point to a most distal end, and extends to be curved outward in the radial direction and toward a proximal side from the most distal end.

11. A balloon catheter comprising:
a shaft that extends in an axial direction;
a distal member that is fused to a distal side of the shaft and is more flexible than the shaft; and
a balloon that is disposed on an outer periphery of the shaft, wherein
the shaft includes
an outer layer and
an inner layer that is disposed inward in a radial direction of the outer layer,
in a cross-sectional view in the axial direction,
a distal portion of the shaft intrudes in an arc outward in the radial direction from an inner surface of the distal member,
an outer peripheral surface of the distal portion of the shaft forms an outer periphery of the balloon catheter, and
a most distal portion of the balloon is positioned inward in the radial direction with respect to an outer surface of the distal portion of the shaft.

12. The balloon catheter according to claim 11, wherein
an inner surface-side proximal portion is provided in a section interposed between the inner surface of the distal member and an inner surface of the shaft in contact with the distal member, the inner surface-side proximal portion having a thickness in the section that increases in the radial direction along a curvature of the inner surface in a distal direction from a site of a lumen of the inner layer of the shaft, and
an outer surface-side proximal portion is provided in a section interposed between an outer surface of the distal member and an outer surface of the shaft, the outer surface-side proximal portion having a thickness in the section that increases in the radial direction along a curvature of an outer surface of the outer layer in the distal direction from a site of the outer surface of the shaft.

13. A medical elongated body comprising:
a catheter shaft that extends in an axial direction and is provided with an outer layer and an inner layer that is disposed inward in a radial direction of the outer layer;
a distal member that is fused to a distal side of the catheter shaft, wherein
in a cross-sectional view in the axial direction,
a distal portion of the outer layer intrudes in an arc inward in the radial direction from an outer surface of the distal member, extends to be curved to a most distal end of the outer layer, is gradually tapered in a thickness, and terminates at an intermediate position in the radial direction of the catheter shaft.

14. The medical elongated body according to claim 13,
wherein a distal portion of the inner layer intrudes in an arc outward in the radial direction from an inner surface of the distal member.
